# EUROPÄISCHE PATENTSCHRIFT

(11) **EP 1 761 517 B1**
(45) Veröffentlichungstag und Bekanntmachung des Hinweises auf die Patenterteilung: **24.09.2014**
(21) Anmeldenummer: 05758460.9
(22) Anmeldetag: 18.06.2005
(51) Int. Cl.: C07D 401/04, A61K 31/4196, A61P 3/10

(54) **NEUE IMIDAZOLE UND TRIAZOLE, DEREN HERSTELLUNG UND VERWENDUNG ALS ARZNEIMITTEL**
NOVEL IMIDAZOLES AND TRIAZOLES, THE PRODUCTION THEREOF, AND THE USE OF THE SAME AS MEDICAMENTS
NOUVEAUX IMIDAZOLES ET TRIAZOLES, PROCEDE POUR LES PRODUIRE ET LEUR UTILISATION EN TANT QUE MEDICAMENTS

(30) Priorität: 24.06.2004 DE 102004030502
(43) Veröffentlichungstag der Anmeldung: 14.03.2007
(73) Patentinhaber: BOEHRINGER INGELHEIM INTERNATIONAL GMBH, 55216 Ingelheim (DE); Boehringer Ingelheim Pharma GmbH & Co. KG, 55216 Ingelheim (DE)
(72) Erfinder: ECKHARDT, Matthias, 88400 Biberach (DE); HIMMELSBACH, Frank, 88441 Mittelbiberach (DE); LANGKOPF, Elke, 88447 Warthausen (DE); THOMAS, Leo, 88400 Biberach (DE); TADAYYON, Mohammad, SG14 1HQ Hertford (GB)
(74) Vertreter: Simon, Elke Anna Maria
(86) Internationale Anmeldenummer: PCT/EP2005/006587
(87) Internationale Veröffentlichungsnummer: WO 2006/000354

(56) Entgegenhaltungen:
- EP-A- 0 399 285
- EP-A- 0 412 358
- EP-A- 0 524 482
- WO-A-2004/050658
- FR-A- 2 707 641

## Beschreibung

Gegenstand der vorliegenden Erfindung sind Verbindungen der allgemeinen Formel 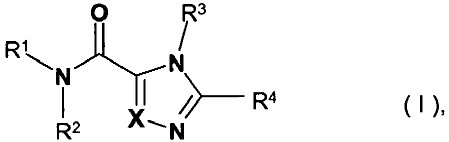 in der
R¹ eine Aryl-C₁₋₆-alkyl- oder Heteroaryl-C₁₋₆-alkylgruppe, in der jede Methylengruppe der Alkylgruppe durch ein oder zwei Fluoratome oder eine C₁₋₃-Alkylgruppe substituiert und eine Methylengruppe durch eine Carbonylgruppe ersetzt sein kann,
oder eine Aryl-C₂₋₆-alkenyl- oder Heteroaryl-C₂₋₆-alkenylgruppe, in denen die Alkenylkette mit 1 bis 10 Fluoratomen oder einer Cyan-, C₁₋₃-Alkyloxy-carbonyl-, C₁₋₃-Alkyl- oder Nitrogruppe substituiert sein kann,
R² ein Wasserstoffatom,
eine C₁₋₆-Alkylgruppe,
eine durch eine Gruppe Rₐ substituierte C₁₋₆-Alkylgruppe, wobei
   Rₐ eine C₁₋₄-Alkoxy-carbonyl-gruppe bedeutet,
eine durch eine Gruppe R_{c} substituierte NH-Gruppe, wobei
   R_{c} eine C₁₋₆-Alkyl-gruppe bedeutet,
eine Hydroxygruppe, oder
eine C₁₋₄-Alkoxygruppe,
X ein Stickstoffatom oder eine CH-Gruppe,
R³ eine 1-Buten-1-yl-, 2-Buten-1-yl-, 3-Methyl-2-buten-1-yl-, 2-Butin-1-yl-, Cyclopent-1-enyl-methyl-, Furanylmethyl-, Thienylmethyl-, Chlorbenzyl-, Brombenzyl-, lodbenzyl-, Methoxybenzyl- oder Cyanbenzylgruppe, und
R⁴ eine N-(2-Aminoethyl)-N-methyl-aminogruppe, die im Ethylteil durch ein oder zwei C₁₋₃-Alkylgruppen substituiert sein kann, oder
eine 3-Aminopiperidin-1-yl-, Piperazin-1-yl- oder [1,4]Diazepan-1-ylgruppe, wobei die vorstehend erwähnten Gruppen jeweils zusätzlich durch ein oder zwei C₁₋₃-Alkylgruppen substituiert sein können, bedeuten,
wobei unter den bei der Definition der vorstehend genannten Reste erwähnten Arylgruppen Phenyl- oder Naphthylgruppen zu verstehen sind, welche unabhängig voneinander durch Rₕ mono-, di- oder trisubstituiert sein können, wobei die Substituenten gleich oder verschieden sein können und Rₕ ein Fluor-, Chlor-, Brom- oder lodatom, eine Trifluormethyl-, Cyan-, Nitro-, Amino-, Aminocarbonyl-, C₁₋₃-Alkoxy-carbonyl-, Aminosulfonyl-, Methylsulfonyl, Acetylamino-, Methylsulfonylamino-, C₁₋₃-Alkyl-, Cyclopropyl-, Ethenyl-, Ethinyl-, Morpholinyl-, Hydroxy-, C₁₋₃-Alkyloxy-, Difluormethoxy- oder Trifluormethoxygruppe darstellt, und in denen zusätzlich jedes Wasserstoffatom durch ein Fluoratom ersetzt sein kann,
unter den bei der Definition der vorstehend erwähnten Reste erwähnten Heteroarylgruppen eine Pyrrolyl-, Furanyl-, Thienyl-, Pyridyl-, Indolyl-, Benzofuranyl-, Benzothiophenyl-, Phenanthridinyl-, Chinolinyl- oder Isochinolinylgruppe zu verstehen ist,
oder eine Pyrrolyl-, Furanyl-, Thienyl- oder Pyridylgruppe zu verstehen ist, in der eine oder zwei Methingruppen durch Stickstoffatome ersetzt sind,
oder eine Indolyl-, Benzofuranyl-, Benzothiophenyl-, Phenanthridinyl-, Chinolinyl- oder Isochinolinylgruppe zu verstehen ist, in der eine bis drei Methingruppen durch Stickstoffatome ersetzt sind,
oder eine 1,2-Dihydro-2-oxo-pyridinyl-, 1,4-Dihydro-4-oxo-pyridinyl-, 2,3-Dihydro-3-oxo-pyridazinyl-, 1,2,3,6-Tetrahydro-3,6-dioxo-pyridazinyl-, 1,2-Dihydro-2-oxo-pyrimidinyl-, 3,4-Dihydro-4-oxo-pyrimidinyl-, 1,2,3,4-Tetrahydro-2,4-dioxo-pyrimidinyl-, 1,2-Dihydro-2-oxo-pyrazinyl-, 1,2,3,4-Tetrahydro-2,3-dioxo-pyrazinyl-, 2,3-Dihydro-2-oxo-indolyl-, 2,3-Dihydrobenzofuranyl-, 2,3-Dihydro-2-oxo-1*H*-benzimidazolyl-, 2,3-Dihydro-2-oxo-benzoxazolyl-, 1,2-Dihydro-2-oxo-chinolinyl-, 1,4-Dihydro-4-oxo-chinolinyl-, 1,2-Dihydro-1-oxo-isochinolinyl-, 1,4-Dihydro-4-oxo-cinnolinyl-, 1,2-Dihydro-2-oxo-chinazolinyl-, 3,4-Dihydro-4-oxo-chinazolinyl-, 1,2,3,4-Tetrahydro-2,4-dioxo-chinazolinyl-, 1,2-Dihydro-2-oxochinoxalinyl-, 1,2,3,4-Tetrahydro-2,3-dioxo-chinoxalinyl-, 1,2-Dihydro-1-oxo-phthalazinyl-, 1,2,3,4-Tetrahydro-1,4-dioxo-phthalazinyl-, Chromanyl-, Cumarinyl-, 2,3-Dihydro-benzo[1,4]dioxinyl- oder 3,4-Dihydro-3-oxo-2*H*-benzo[1,4]oxazinyl-Gruppe zu verstehen ist,
   und die vorstehend erwähnten Heteroarylgruppen durch Rₕ mono- oder disubstituiert sein können, wobei die Substituenten gleich oder verschieden sein können und Rₕ wie vorstehend erwähnt definiert ist,
wobei, soweit nichts anderes erwähnt wurde, die vorstehend erwähnten Alkyl- und Alkenyl-gruppen geradkettig oder verzweigt sein können,
deren Enantiomere, deren Diastereomere, deren Gemische und deren Salze.

Weitere Gegenstände der vorliegenden Erfindung sind den vorliegenden Ansprüchen zu entnehmen.

Weiterer Gegenstand der vorliegenden Beschreibung sind substituierte Imidazole und Triazole der allgemeinen Formel 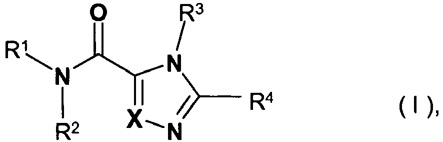 deren Tautomere, deren Stereoisomere, deren Gemische und deren Salze, insbesondere deren physiologisch verträgliche Salze mit anorganischen oder organischen Säuren oder Basen, welche wertvolle pharmakologische Eigenschaften aufweisen, insbesondere eine Hemmwirkung auf die Aktivität des Enzyms Dipeptidylpeptidase-IV (DPP-IV), deren Herstellung, deren Verwendung zur Prävention oder Behandlung von Krankheiten oder Zuständen, die in Zusammenhang mit einer erhöhten DPP-IV Aktivität stehen oder die durch Reduktion der DPP-IV Aktivität verhindert oder gemildert werden können, insbesondere von Diabetes mellitus Typ I oder Typ II, die eine Verbindung der allgemeinen Formel (I) oder ein physiologisch verträgliches Salz davon enthaltenden Arzneimittel sowie Verfahren zu deren Herstellung.

In den europäischen Anmeldungen EP 332 991, EP 399 285 und EP 412 358 werden Triazole als Pestizide beschrieben.

In der obigen Formel I bedeuten
R¹ eine Aryl-C₁₋₆-alkyl- oder Heteroaryl-C₁₋₆-alkylgruppe, in der jede Methylengruppe der Alkylgruppe durch ein oder zwei Fluoratome oder eine C₁₋₃-Alkylgruppe substituiert und eine Methylengruppe durch eine Carbonylgruppe ersetzt sein kann,
oder eine Aryl-C₂₋₆-alkenyl- oder Heteroaryl-C₂₋₆-alkenylgruppe, in denen die Alkenylkette mit 1 bis 10 Fluoratomen oder einer Cyan-, C₁₋₃-Alkyloxy-carbonyl-, C₁₋₃-Alkyl- oder Nitrogruppe substituiert sein kann,
R² ein Wasserstoffatom,
eine C₁₋₆-Alkylgruppe,
eine durch eine Gruppe Rₐ substituierte C₁₋₆-Alkylgruppe, wobei
Rₐ ein Fluor-, Chlor- oder Bromatom,
oder eine Trifluormethyl-, Nitro-, Aryl-, Heteroaryl-, Cyano-, Carboxy-, C₁₋₄-Alkoxy-carbonyl-, Aminocarbonyl-, C₁₋₄-Alkylamino-carbonyl-, Di-(C₁₋₃-alkyl)-amino-carbonyl-, Pyrrolidin-1-ylcarbonyl-, Piperidin-1-ylcarbonyl-, Morpholin-4-ylcarbonyl-, Piperazin-1-ylcarbonyl-, 4-(C₁₋₄-Alkyl)-piperazin-1-ylcarbonyl-, C₁₋₄-Alkylcarbonyl-, Arylcarbonyl-, Heteroarylcarbonyl-, C₁₋₃-Alkylsulfinyl- oder C₁₋₃-Alkylsulfonylgruppe bedeutet,
eine ab Position 2 durch eine Gruppe R_{b} substituierte C₂₋₆-Alkylgruppe, wobei
R_{b} eine Hydroxy-, C₁₋₃-Alkoxy-, Amino-, C₁₋₃-Alkylamino-, Di-(C₁₋₃-alkyl)-amino-, Pyrrolidin-1-yl-, Piperidin-1-yl-, Morpholin-4-yl-, Piperazin-1-yl- oder 4-(C₁₋₃-Alkyl)-piperazin-1-ylgruppe bedeutet,
eine durch eine Gruppe R_{c} substituierte NH-Gruppe, wobei
R_{c} eine C₁-₆-Alkyl-, C₂₋₆-Alkenyl-, Aryl-, Heteroaryl-, C₁₋₄-Alkoxy-carbonyl-, Aminocarbonyl-, C₁₋₄-Alkylamino-carbonyl-, Di-(C₁₋₃-alkyl)-amino-carbonyl-, Pyrrolidin-1-ylcarbonyl-, Piperidin-1-ylcarbonyl-, Morpholin-4-ylcarbonyl-, Piperazin-1-ylcarbonyl-, 4-(C₁₋₄-Alkyl)-piperazin-1-ylcarbonyl-, C₁-₄-Alkylcarbonyl-, Arylcarbonyl-, Heteroarylcarbonyl-, C₁₋₃-Alkylsulfinyl oder C₁₋₃-Alkylsulfonylgruppe bedeutet,
eine Hydroxygruppe,
eine C₁₋₄-Alkoxygruppe,
oder eine C₂₋₆-Alkenyl- oder C₂₋₆-Alkinylgruppe,
X ein Stickstoffatom oder eine CH-Gruppe,
R³ eine gegebenenfalls durch eine C₁₋₃-Alkylgruppe substituierte C₅₋₇-Cyclo-alkenylmethylgruppe,
eine Arylmethyl- oder Heteroarylmethylgruppe,
eine geradkettige oder verzweigte C₂-₈-Alkenylgruppe, die durch 1 bis 15 Fluoratome oder eine Cyano-, Nitro- oder C₁₋₃-Alkoxy-carbonylgruppe substituiert sein kann,
oder eine geradkettige oder verzweigte C₃₋₆-Alkinylgruppe, die durch 1 bis 9 Fluoratome oder eine Cyano-, Nitro- oder C₂₋₈-Alkoxy-carbonylgruppe substituiert sein kann,
und
R⁴ eine Pyrrolidin-1-yl- oder Azetedin-1-ylgruppe, die in 3-Stellung durch eine Amino- oder C₁₋₃-Alkylaminogruppe substituiert ist und zusätzlich durch eine oder zwei C₁₋₃-Alkylgruppen substituiert sein kann,
eine Piperidin-1-yl- oder Hexahydroazepin-1-ylgruppe, die in 3-Stellung oder in 4-Stellung durch eine Amino-Gruppe oder C₁₋₃-Alkylaminogruppe substituiert ist und zusätzlich durch eine oder zwei C₁₋₃-Alkylgruppen substituiert sein kann,
eine Piperazin-1-yl- oder Homopiperazin-1-ylgruppe, die durch eine oder zwei C₁₋₃-Alkylgruppen substituiert sein kann,
eine durch die Reste R¹⁵ und R¹⁶ substituierte Aminogruppe, in der
R¹⁵ ein Wasserstoffatom, eine C₁₋₆-Alkyl-, C₃₋₆-Cycloalkyl-, C₃₋6-Cycloalkyl-C₁₋₃-alkyl-, Aryl- oder Aryl-C₁₋₃-alkylgruppe und
R¹⁶ eine R¹⁷-C₂₋₃-alkylgruppe darstellt, wobei der C₂₋₃-Alkylteil geradkettig ist und durch 1 bis 4 C₁₋₃-Alkylgruppen, die gleich oder verschieden sein können, substituiert sein kann und wobei die C₂₋₃-Alkylgruppe ab Position 2 mit R¹⁷ verknüpft sein kann, und
R¹⁷ eine Amino- oder C₁₋₃-Alkylaminogruppe darstellt,
eine durch die Reste R¹⁵ und R¹⁸ substituierte Aminogruppe, in der
R¹⁵ wie vorstehend erwähnt definiert ist und R¹⁸ eine in 1-Stellung des Cycloalkylrestes durch R¹⁹ substituierte C₃₋₁₀-Cycloalkyl-C₁₋₂-alkyl-gruppe oder eine in 1- oder 2-Stellung durch eine R¹⁹-C₁₋₂-alkyl-gruppe substituierte C₃₋₁₀-Cycloalkylgruppe darstellt, wobei R¹⁹ eine Amino- oder C₁₋₃-Alkylaminogruppe darstellt,
eine durch die Reste R¹⁵ und R²⁰ substituierte Aminogruppe, in der
R¹⁵ wie vorstehend erwähnt definiert ist und R²⁰ eine C₄₋₁₀-Cycloalkyl- oder C₄₋₁₀-Cycloalkyl-methyl-gruppe, in der eine Methylengruppe der C₄₋₁₀-Cycloalkylgruppe durch eine -NH- Gruppe ersetzt ist,
oder eine durch die Reste R¹⁵ und R²¹ substituierte Aminogruppe, in der
R¹⁵ wie vorstehend erwähnt definiert ist und R²¹ eine in 2- oder 3-Stellung durch eine Amino- oder C₁₋₃-Alkylaminogruppe substituierte C₃₋₁₀-Cycloalkylgruppe darstellt,
wobei die vorstehend genannten Reste R¹⁸, R²⁰ und R²¹ durch R_{d} mono- oder disubstituiert sein können, wobei die Substituenten gleich oder verschieden sein können und R_{d} ein Fluoratom, eine C₁₋₃-Alkyl-, Trifluormethyl-, Cyan-, Amino-, C₁₋₃-Alkylamino-, Hydroxy- oder C₁₋₃-Alkyloxygruppe darstellt, und in denen ein oder zwei Methylengruppen des Cycloalkylrests unabhängig voneinander jeweils durch ein Sauerstoff- oder Schwefelatom oder durch eine -NH- oder -N(C₁₋₃-Alkyl)- Gruppe, oder durch eine Carbonyl-, Sulfinyl- oder Sulfonylgruppe ersetzt sein können,
wobei unter den bei der Definition der vorstehend genannten Reste erwähnten Arylgruppen Phenyl- oder Naphthylgruppen zu verstehen sind, welche unabhängig voneinander durch Rₕ mono-, di- oder trisubstituiert sein können, wobei die Substituenten gleich oder verschieden sein können und Rₕ ein Fluor-, Chlor-, Brom- oder lodatom, eine Trifluormethyl-, Cyan-, Nitro-, Amino-, Aminocarbonyl-, C₁₋₃-Alkoxy-carbonyl-, Aminosulfonyl-, Methylsulfonyl, Acetylamino-, Methylsulfonylamino-, C₁₋₃-Alkyl-, Cyclopropyl-, Ethenyl-, Ethinyl-, Morpholinyl-, Hydroxy-, C₁₋₃-Alkyloxy-, Difluormethoxy- oder Trifluormethoxygruppe darstellt, und in denen zusätzlich jedes Wasserstoffatom durch ein Fluoratom ersetzt sein kann,
unter den bei der Definition der vorstehend erwähnten Reste erwähnten Heteroarylgruppen eine Pyrrolyl-, Furanyl-, Thienyl-, Pyridyl-, Indolyl-, Benzofuranyl-, Benzothiophenyl-, Phenanthridinyl-, Chinolinyl- oder Isochinolinylgruppe zu verstehen ist,
oder eine Pyrrolyl-, Furanyl-, Thienyl- oder Pyridylgruppe zu verstehen ist, in der eine oder zwei Methingruppen durch Stickstoffatome ersetzt sind,
oder eine Indolyl-, Benzofuranyl-, Benzothiophenyl-, Phenanthridinyl-, Chinolinyl- oder Isochinolinylgruppe zu verstehen ist, in der eine bis drei Methingruppen durch Stickstoffatome ersetzt sind,
oder eine 1,2-Dihydro-2-oxo-pyridinyl-, 1,4-Dihydro-4-oxo-pyridinyl-, 2,3-Dihydro-3-oxo-pyridazinyl-, 1,2,3,6-Tetrahydro-3,6-dioxo-pyridazinyl-, 1,2-Dihydro-2-oxo-pyrimidinyl-, 3,4-Dihydro-4-oxo-pyrimidinyl-, 1,2,3,4-Tetrahydro-2,4-dioxo-pyrimidinyl-, 1,2-Dihydro-2-oxo-pyrazinyl-, 1,2,3,4-Tetrahydro-2,3-dioxo-pyrazinyl-, 2,3-Dihydro-2-oxo-indolyl-, 2,3-Dihydrobenzofuranyl-, 2,3-Dihydro-2-oxo-1*H*-benzimidazolyl-, 2,3-Dihydro-2-oxo-benzoxazolyl-, 1,2-Dihydro-2-oxo-chinolinyl-, 1,4-Dihydro-4-oxo-chinolinyl-, 1,2-Dihydro-1-oxo-isochinolinyl-, 1,4-Dihydro-4-oxo-cinnolinyl-, 1,2-Dihydro-2-oxo-chinazolinyl-, 3,4-Dihydro-4-oxo-chinazolinyl-, 1,2,3,4-Tetrahydro-2,4-dioxo-chinazolinyl-, 1,2-Dihydro-2-oxochinoxalinyl-, 1,2,3,4-Tetrahydro-2,3-dioxo-chinoxalinyl-, 1,2-Dihydro-1-oxo-phthalazinyl-, 1,2,3,4-Tetrahydro-1,4-dioxo-phthalazinyl-, Chromanyl-, Cumarinyl-, 2,3-Dihydro-benzo[1,4]dioxinyl-oder 3,4-Dihydro-3-oxo-2*H*-benzo[1,4]oxazinyl-Gruppe zu verstehen ist,
und die vorstehend erwähnten Heteroarylgruppen durch Rₕ mono- oder disubstituiert sein können, wobei die Substituenten gleich oder verschieden sein können und Rₙ wie vorstehend erwähnt definiert ist,
unter den bei der Definition der vorstehend erwähnten Cycloalkylreste sowohl monocyclische als auch polycyclische Ringsysteme zu verstehen sind, wobei die Mehrfachcyclen anelliert, spiro-verknüpft oder verbrückt aufgebaut sein können, beispielsweise sind unter Mehrfachcyclen Decalin, Oktahydroinden, Norbornan, Spiro[4.4]nonan, Spiro[4.5]dekan, Bicyclo[2.1.1]hexan, Bicyclo[2.2.2]octan, Bicyclo[3.2.1]octan, Bicyclo[3.2.2]nonan, Bicyclo[3.3.1]nonan, Bicyclo[3.3.2]dekan oder Adamantan zu verstehen,
wobei, soweit nichts anderes erwähnt wurde, die vorstehend erwähnten Alkyl-, Alkenyl- und Alkinylgruppen geradkettig oder verzweigt sein können,
deren Tautomere, Enantiomere, Diastereomere, deren Gemische, deren Prodrugs und deren Salze.

Die bei der Definition der vorstehend erwähnten Reste erwähnten Carboxygruppen können durch eine in-vivo in eine Carboxygruppe überführbare Gruppe oder durch eine unter physiologischen Bedingungen negativ geladene Gruppe ersetzt sein,
des Weiteren können die bei der Definition der vorstehend erwähnten Reste erwähnten Amino- und Iminogruppen durch einen in-vivo abspaltbaren Rest substituiert sein. Derartige Gruppen werden beispielsweise in der WO 98/46576 und von N.M. Nielsen et al. in International Journal of Pharmaceutics 39, 75-85 (1987) beschrieben.

Unter einer in-vivo in eine Carboxygruppe überführbare Gruppe ist beispielsweise eine Hydroxymethylgruppe, eine mit einem Alkohol veresterte Carboxygruppe, in der der alkoholische Teil vorzugsweise ein C₁₋₆-Alkanol, ein Phenyl-C₁₋₃-alkanol, ein C₃₋₉-Cycloalkanol, wobei ein C₅₋₈-Cycloalkanol zusätzlich durch ein oder zwei C₁₋₃-Alkylgruppen substituiert sein kann, ein C₅₋₈-Cycloalkanol, in dem eine Methylengruppe in 3- oder 4-Stellung durch ein Sauerstoffatom oder durch eine gegebenenfalls durch eine C₁₋₃-Alkyl-, Phenyl-C₁₋₃-alkyl-, Phenyl-C₁₋₃-alkoxy-carbonyl- oder C₂₋₆-Alkanoylgruppe substituierte Iminogruppe ersetzt ist und der Cycloalkanolteil zusätzlich durch ein oder zwei C₁₋₃-Alkylgruppen substituiert sein kann, ein C₄₋₇-Cycloalkenol, ein C₃₋₅-Alkenol, ein Phenyl-C₃₋₅-alkenol, ein C₃₋₅-Alkinol oder Phenyl-C₃₋₅-alkinol mit der Maßgabe, daß keine Bindung an das Sauerstoffatom von einem Kohlenstoffatom ausgeht, welches eine Doppel- oder Dreifachbindung trägt, ein C₃₋₈-Cycloalkyl-C₁₋₃-alkanol, ein Bicycloalkanol mit insgesamt 8 bis 10 Kohlenstoffatomen, das im Bicycloalkylteil zusätzlich durch eine oder zwei C₁-₃-Alkylgruppen substituiert sein kann, ein 1,3-Dihydro-3-oxo-1-isobenzfuranol oder ein Alkohol der Formel

Rₚ-CO-O-(R_{q}CRᵣ)-OH,

in dem
Rₚ eine C₁₋₈-Alkyl-, C₅₋₇-Cycloalkyl-, C₁₋₈-Alkyloxy-, C₅₋₇-Cycloalkyloxy-, Phenyl- oder Phenyl- C₁₋₃-alkylgruppe,
Rq ein Wasserstoffatom, eine C₁₋₃-Alkyl-, C₅₋₇-Cycloalkyl- oder Phenylgruppe und
Rᵣ ein Wasserstoffatom oder eine C₁-₃-Alkylgruppe darstellen,
unter einer unter physiologischen Bedingungen negativ geladenen Gruppe wie eine Tetrazol-5-yl-, Phenylcarbonylaminocarbonyl-, Trifluormethylcarbonylaminocarbonyl-, C₁₋₆-Alkylsulfonylamino-, Phenylsulfonylamino-, Benzylsulfonylamino-, Trifluormethylsulfonylamino-, C₁₋₆-Alkylsulfonylaminocarbonyl-, Phenylsulfonylaminocarbonyl-, Benzylsulfonylaminocarbonyl- oder Perfluor-C₁₋₆-alkylsulfonylamino-carbonylgruppe
und unter einem von einer Imino- oder Aminogruppe in-vivo abspaltbaren Rest beispielsweise eine Hydroxygruppe, eine Acylgruppe wie eine gegebenenfalls durch Fluor-, Chlor-, Brom- oder Jodatome, durch C₁₋₃-Alkyl- oder C₁₋₃-Alkoxygruppen mono- oder disubstituierte Phenylcarbonylgruppe, wobei die Substituenten gleich oder verschieden sein können, eine Pyridinoylgruppe oder eine C₁₋₁₆-Alkanoylgruppe wie die Formyl-, Acetyl-, Propionyl-, Butanoyl-, Pentanoyl- oder Hexanoylgruppe, eine 3,3,3-Trichlorpropionyl- oder Allyloxycarbonylgruppe, eine C₁-₁₆-Alkoxycarbonyl- oder C₁₋₁₆-Alkylcarbonyloxygruppe, in denen Wasserstoffatome ganz oder teilweise durch Fluor- oder Chloratome ersetzt sein können, wie die Methoxycarbonyl-, Ethoxycarbonyl-, Propoxycarbonyl-, Isopropoxycarbonyl-, Butoxycarbonyl-, tert.-Butoxycarbonyl-, Pentoxycarbonyl-, Hexoxycarbonyl-, Octyloxycarbonyl-, Nonyloxycarbonyl-, Decyloxycarbonyl-, Undecyloxycarbonyl-, Dodecyloxycarbonyl-, Hexadecyloxycarbonyl-, Methylcarbonyloxy-, Ethylcarbonyloxy-, 2,2,2-Trichlorethylcarbonyloxy-, Propylcarbonyloxy-, Isopropylcarbonyloxy-, Butylcarbonyloxy-, tert.Butylcarbonyloxy-, Pentylcarbonyloxy-, Hexylcarbonyloxy-, Octylcarbonyloxy-, Nonylcarbonyloxy-, Decylcarbonyloxy-, Undecylcarbonyloxy-, Dodecylcarbonyloxy- oder Hexadecylcarbonyloxygruppe, eine Phenyl-C₁₋₆-alkoxycarbonylgruppe wie die Benzyloxycarbonyl-, Phenylethoxycarbonyl- oder Phenylpropoxycarbonylgruppe, eine 3-Amino-propionylgruppe, in der die Aminogruppe durch C₁₋₆-Alkyl- oder C₃₋₇-Cycloalkylgruppen mono- oder disubstituiert und die Substituenten gleich oder verschieden sein können, eine C₁₋₃-Alkylsulfonyl-C₂₋₄-alkoxycarbonyl-, C₁₋₃-Alkoxy-C₂₋₄-alkoxy-C₂₋₄-alkoxycarbonyl-, Rₚ-CO-O-(R_{q}CRᵣ)-O-CO-, C₁₋₆-Alkyl-CO-NH- (RₛCRₜ)-O-CO- oder C₁-₆-Alkyl-CO-O-(RₛCRₜ)-(RₛCRₜ)-O-CO-Gruppe, in denen Rₚ bis Rᵣ wie vorstehend erwähnt definiert sind,
Rₛ und Rₜ, die gleich oder verschieden sein können, Wasserstoffatome oder C₁₋₃-Alkylgruppen darstellen,
zu verstehen.

Des Weiteren schließen die in den vor- und nachstehenden Definitionen erwähnten gesättigten Alkyl- und Alkoxyteile, die mehr als 2 Kohlenstoffatome enthalten, soweit nichts Anderes erwähnt wurde, auch deren verzweigte Isomere wie beispielsweise die Isopropyl-, tert.Butyl-, Isobutylgruppe etc. ein.

Für R¹ kommt beispielsweise die Bedeutung einer 2-Cyanbenzyl-, 3-Fluorbenzyl-, 3-Methoxybenzyl-, 4-Brom-2-cyanbenzyl, 3-Chlor-2-cyanbenzyl, 2-Cyan-4-fluorbenzyl, 3,5-Dimethoxybenzyl-, 2,6-Dicyanbenzyl-, 5-Cyanfuranylmethyl-, Oxazolylmethyl-, Isoxazolylmethyl-, 5-Methoxycarbonylthienylmethyl-, Pyridinylmethyl-, 3-Cyanpyridin-2-ylmethyl-, 6-Cyanpyridin-2-ylmethyl-, 6-Fluorpyridin-2-ylmethyl-, Pyrimidin-2-yl-methyl-, 4-Methyl-pyrimidin-2-ylmethyl-, 4,6-Dimethyl-pyrimidin-2-ylmethyl-, 3-(2-Cyanphenyl)-prop-2-enyl-, 3-(Pyridin-2-yl)-prop-2-enyl-, 3-(Pentafluorphenyl)-prop-2-enyl-, Phenylcarbonylmethyl-, 3-Methoxyphenylcarbonylmethyl-, Naphth-1-ylmethyl-, 4-Cyannaphth-1-ylmethyl-, Chinolin-1-ylmethyl-, 4-Cyanchinolin-1-ylmethyl-, Iso-chinolin-1 -ylmethyl-, 4-Cyanisochinolin-1-ylmethyl-, 3-Methylisochinolin-1-ylmethyl-, Chinazolin-2-ylmethyl-, 4-Methylchinazolin-2-ylmethyl-, [1,5]Naphthiridin-2-ylmethyl-, [1,5]Naphthiridin-3-ylmethyl-, Phenanthridin-6-ylmethyl-, Chinoxalin-6-ylmethyl- oder 2,3-Dimethyl-chinoxalin-6-ylmethylgruppe in Betracht.

Für R² kommt beispielsweise die Bedeutung eines Wasserstoffatoms, einer Methyl-, Ethyl-, Propyl-, Butyl-, 2-Propen-1-yl-, 2-Propin-1-yl-, Benzyl-, 2-Phenylethyl-, Phenylcarbonylmethyl-, 3-Phenylpropyl-, Hydroxy-, 2-Hydroxyethyl-, 2-Methoxyethyl-, 2-Ethoxyethyl-, 2-(Dimethylamino)ethyl-, 2-(Diethylamino)ethyl-, 3-Hydroxypropyl-, 3-Methoxypropyl-, 3-(Dimethylamino)propyl-, 3-(Pyrrolidino)propyl-, 3-(Piperidino)propyl-, 3-(Morpholino)propyl-,3-(Piperazino)propyl-, 3-(4-Methylpiperazino)-propyl-, Carboxymethyl-, (Methoxycarbonyl)methyl-, (Ethoxycarbonyl)methyl-, 2-Carboxyethyl-, 2-(Methoxycarbonyl)ethyl-, 2-(Ethoxycarbonyl)ethyl-, 3-Carboxypropyl-, 3-(Methoxycarbonyl)propyl-, 3-(Ethoxycarbonyl)propyl-, (Aminocarbonyl)-methyl-, (Methylaminocarbonyl)methyl-, (Dimethylaminocarbonyl)methyl-, (Pyrrolidinocarbonyl)methyl-, (Piperidinocarbonyl)methyl-, (Morpholinocarbonyl)methyl-, 2-(Aminocarbonyl)ethyl-, 2-(Methylaminocarbonyl)ethyl-, 2-(Dimethylaminocarbonyl)-ethyl-, 2-(Pyrrolidinocarbonyl)ethyl-, 2-(Piperidinocarbonyl)ethyl-, 2-(Morpholinocarbonyl)ethyl-, Phenylcarbonylmethyl-, Cyanmethyl-, 2-Cyanethyl-, Pyridin-3-yl-methyl-, Pyridin-4-ylmethyl-, Methylamino-, Etrhylamino-, Methoxycarbonylamino-, Ethoxycarbonylamino-, Methylcarbonylamino-, Ethylcarbonylamino-, Aminocarbonylamino-, Methylaminocarbonylamino-, Dimethylaminocarbonylamino-, Methylsulfonylamino-, Phenylamino- oder Pyridin-2-ylaminogruppe in Betracht.

Für R³ kommt beispielsweise die Bedeutung einer 2-Propen-1-yl-, 2-Methyl-2-propen-1-yl-, 1-Buten-1-yl-, 2-Buten-1-yl-, 3-Buten-1-yl-, 2-Methyl-2-buten-1-yl-, 3-Methyl-2-buten-1-yl-, 2,3-Dimethyl-2-buten-1-yl-, 3-Methyl-3-buten-1-yl-, 1-Cyclopenten-1-ylmethyl-, (2-Methyl-1-cyclopenten-1-yl)methyl-, 1-Cyclohexen-1-ylmethyl-, 2-Propin-1-yl-, 2-Butin-1-yl, 3-Butin-1-yl, 2-Chlorbenzyl-, 2-Brombenzyl-, 2-lodbenzyl-, 2-Cyanbenzyl-, 3-Fluorbenzyl-, 2-Methoxybenzyl-, 2-Furanylmethyl, 3-Furanylmethyl-, 2-Thienylmethyl- oder 3-Thienylmethylgruppe in Betracht.

Für R⁴ kommt beispielsweise die Bedeutung einer 3-Aminopyrrolidin-1-yl-, 3-Amino-piperidin-1-yl-, 3-(Methylamino)-piperidin-1-yl-, 3-(Ethylamino)-piperidin-1-yl-, 3-Amino-2-methyl-piperidin-1-yl-, 3-Amino-3-methyl-piperidin-1-yl-, 3-Amino-4-methylpiperidin-1-yl-, 3-Amino-5-methyl-piperidin-1-yl-, 3-Amino-6-methyl-piperidin-1-yl-, 4-Aminopiperidin-1-yl-, 3-Amino-hexahydroazepin-1-yl-, 4-Amino-hexahydroazepin-1-yl-, (2-Aminocyclopropyl)amino-, (2-Aminocyclobutyl)amino-, (3-Aminocyclobutyl)-amino-, (2-Aminocyclopentyl)amino-, (3-Aminocyclopentyl)amino-, (2-Aminocyclohexyl)amino-, (3-Aminocyclohexyl)amino-, Piperazin-1-yl-, Homopiperazin-1-yl, N-(2-Aminoethyl)-N-methylamino-, N-(2-Aminopropyl)-N-methylamino- oder N-(2-Amino-2-methyl-propyl)-N-methylaminogruppe in Betracht.

Bevorzugte Verbindungen der obigen allgemeinen Formel I sind diejenigen, in denen
R¹ und R² wie oben erwähnt definiert sind,
X ein Stickstoffatom oder eine CH-Gruppe,
R³ eine 1-Buten-1-yl-, 2-Buten-1-yl-, 3-Methyl-2-buten-1-yl-, 2-Butin-1-yl-, Cyclopent-1-enyl-methyl-, Furanylmethyl-, Thienylmethyl-, Chlorbenzyl-, Brombenzyl-, lodbenzyl-, Methoxybenzyl- oder Cyanbenzylgruppe, und
R⁴ eine N-(2-Aminoethyl)-N-methyl-aminogruppe, die im Ethylteil durch ein oder zwei C₁₋₃-Alkylgruppen substituiert sein kann, oder
eine 3-Aminopiperidin-1-yl-, Piperazin-1-yl- oder [1,4]Diazepan-1-ylgruppe, wobei die vorstehend erwähnten Gruppen jeweils zusätzlich durch ein oder zwei C₁₋₃-Alkylgruppen substituiert sein können, bedeuten,
deren Enantiomere, deren Diastereomere, deren Gemische und deren Salze.

Besonders bevorzugte Verbindungen der obigen allgemeinen Formel I sind diejenigen, in denen
R¹ eine Phenylmethyl-, Phenylcarbonylmethyl-, Phenylprop-2-enyl-, Pyridinylmethyl-, Pyrimidinylmethyl-, Naphthylmethyl-, Chinolinylmethyl-, Isochinolinylmethyl-, Chinazolinylmethyl-, Chinoxalinylmethyl-, Phenanthridinylmethyl-, Naphthyridinylmethyl- oder Benzotriazolylmethylgruppe, wobei alle vorstehend genannten Aryl- und Heteroarylgruppen durch ein oder zwei Fluor-, Chlor- oder Bromatome oder ein oder zwei Cyan-, Nitro-, Amino-, C₁₋₃-Alkyl-, C₁₋₃-Alkyloxy- oder Morpholinylgruppen substituiert sein können, wobei die Substituenten gleich oder verschieden sind,
R² ein Wasserstoffatom oder eine Methylgruppe,
X ein Stickstoffatom oder eine CH-Gruppe,
R³ eine 1-Buten-1-yl-, 2-Buten-1-yl-, 3-Methyl-2-buten-1-yl-, 2-Butin-1-yl-, Cyclopent-1-enyl-methyl-, Furanylmethyl-, Thienylmethyl-, Benzyl-, Chlorbenzyl-, Brombenzyl-, lodbenzyl- oder Cyanbenzylgruppe und
R⁴ eine N-(2-Aminoethyl)-N-methylamino-, N-(2-Aminopropyl)-N-methyl-amino-, 3-Aminopiperidin-1-yl-, Piperazin-1-yl-oder [1,4]Diazepan-1-ylgruppe bedeuten,
deren Enantiomere, deren Diastereomere, deren Gemische und deren Salze.

Ganz besonders bevorzugte Verbindungen der obigen allgemeinen Formel I sind diejenigen, in denen
R¹ eine Phenylethyl-, Naphthylmethyl-, Methylisochinolinylmethyl-, Chinolinylmethyl- oder Phenanthridinylmethylgruppe,
R² ein Wasserstoffatom oder eine Methylgruppe,
X ein Stickstoffatom oder eine CH-Gruppe,
R³ eine 3-Methyl-2-buten-1-yl-, 2-Butin-1-yl-, Benzyl- oder 2-Chlorbenzylgruppe und
R⁴ eine 3-Aminopiperidin-1-yl-, [1,4]Diazepan-1-yl- oder Piperazin-1-ylgruppe bedeuten,
deren Enantiomere, deren Diastereomere, deren Gemische und Salze.

Insbesondere sind diejenigen Verbindungen der allgemeinen Formel I bevorzugt, in denen R¹ bis R³ und X wie eingangs erwähnt definiert sind und R⁴ eine 3-Amino-piperidin-1-ylgruppe bedeutet, deren Tautomere, deren Enantiomere, deren Diastereomere, deren Gemische und Salze.

Beispielsweise seien folgende bevorzugte Verbindungen erwähnt:
(1) 1-(But-2-inyl)-2-(3-amino-piperidin-1-yl)-5-(naphth-1-ylmethylaminocarbonyl)-1 H-imidazol
(2) 1-(But-2-inyl)-2-(3-amino-piperidin-1-yl)-5-[N-(naphth-1-ylmethyl)-N-methyl-aminocarbonyl]-1 H-imidazol
(3) 1-(But-2-inyl)-2-(3-amino-piperidin-1-yl)-5-(phenanthridin-6-ylmethylamino-carbonyl)-1 H-imidazol
(4) 1-(But-2-inyl)-2-(3-amino-piperidin-1-yl)-5-(3-methyl-isochinolin-1-ylmethylamino-carbonyl)-1 H-imidazol
(5) 1-(But-2-inyl)-2-(3-amino-piperidin-1-yl)-5-(chinolin-3-ylmethylaminocarbonyl)-1H-imidazol
(6) 1-(But-2-inyl)-2-(3-amino-piperidin-1-yl)-5-(2-phenyl-ethylaminocarbonyl)-1 H-imidazol
(7) 3-(Piperazin-1-yl)-4-(3-methyl-but-2-enyl)-5-(naphth-1-ylmethylaminocarbonyl)-4H-(1,2,4]triazol
(8) 3-(Piperazin-1-yl)-4-benzyl-5-(naphth-1-ylmethylaminocarbonyl)-4H-[1,2,4]triazol
(9) 3-([1,4]Diazepan-1-yl)-4-(3-methyl-but-2-enyl)-5-(naphth-1-ylmethylamino-carbonyl)-4H-[1,2,4]triazol
(10) 3-(3-Amino-piperidin-1-yl)-4-(3-methyl-but-2-enyl)-5-(naphth-1-ylmethylamino-carbonyl)-4H-[1,2,4]triazol
(11) 3-(3-Amino-piperidin-1-yl)-4-benzyl-5-(naphth-1-ylmethylaminocarbonyl)-4H-[1,2,4]triazol
(12) 3-(3-Amino-piperidin-1-yl)-4-(2-chlor-benzyl)-5-(naphth-1-ylmethylamino-carbonyl)-4H-[1,2,4]triazol
(13) 3-(3-Amino-piperidin-1-yl)-4-(but-2-inyl)-5-(3-methyl-isochinolin-1-ylmethyl-aminocarbonyl)-4H-[1,2,4]triazol
(14) 3-(3-Amino-piperidin-1-yl)-4-(but-2-inyl)-5-(chinolin-6-ylmethylaminocarbonyl)-4H-[1,2,4]triazol
(15) 3-(3-Amino-piperidin-1-yl)-4-(but-2-inyl)-5-(phenanthridin-6-ylmethylamino-carbonyl)-4H-[1,2,4]triazol
und deren Salze.

Eine bevorzugte Untergruppe sind diejenigen wie oben definierten Verbindungen der allgemeinen Formel I, in denen X ein Stickstoffatom bedeutet.

Eine zweite bevorzugte Untergruppe stellen diejenigen wie oben definierten Verbindungen der allgemeinen Formel I dar, in denen X eine CH-Gruppe bedeutet.

Erfindungsgemäß erhält man die Verbindungen der allgemeinen Formel I nach an sich bekannten Verfahren, beispielsweise nach folgenden Verfahren:
a) zur Herstellung einer Verbindung der allgemeinen Formel I, in der X ein Stickstoffatom ist: Umsetzung einer Verbindung der allgemeinen Formel 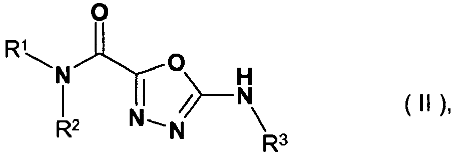 in der R¹ bis R³ wie eingangs erwähnt definiert sind, mit einer Verbindung der allgemeinen Formel

   H - R⁴ (III),

   in der
   R⁴ wie eingangs erwähnt definiert ist.
   Die Umsetzung wird zweckmäßigerweise in einem hoch siedenden Lösungsmittel wie Butanol, Mesitylen, Chlorbenzol, Dimethylsulfoxid, Ethylenglycoldiethylether oder Sulfolan gegebenenfalls in Gegenwart einer anorganischen oder organischen Base, wie z.B. Natriumcarbonat oder Kaliumhydroxid bzw. N-Ethyl-diisopropylamin (Hünig-Base), wobei diese organischen Basen gleichzeitig auch als Lösungsmittel dienen können, bei Temperaturen zwischen 80 und 200°C durchgeführt. Die Umsetzung wird bevorzugt ohne Lösungsmittel in einem Überschuß der eingesetzten Verbindung der allgemeinen Formel III bei 150-200°C durchgeführt, wobei das Heizen in einem Mikrowellenofen gegenüber dem konventionellem bevorzugt wird.
b) zur Herstellung einer Verbindung der allgemeinen Formel I, in der X eine CH-Gruppe ist: Diazotierung mit anschließender Reduktion einer Verbindung der allgemeinen Formel 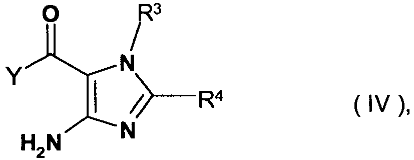 in der Y ein substituiertes Sauerstoff- oder Stickstoffatom, beispielsweise eine C₁₋₆-Alkoxy-, Di-(C₁₋₄-alkyl)-amino- oder -NR¹R²- Gruppe, in der R¹ wie eingangs erwähnt definiert ist und R² mit Ausnahme des Wasserstoffatoms wie eingangs erwähnt definiert ist, bedeutet und R³ und R⁴ wie eingangs erwähnt definiert sind, und gegebenenfalls Umwandlung der Gruppe Y in die Gruppe -NR¹R², in der R¹ und R² wie eingangs erwähnt definiert sind.
   Die Diazotierung wird mit anorganischen oder organischen Nitriten, wie z.B. Natriumnitrit oder Isoamylnitrit, in Gegenwart einer Säure, wie z.B. Salzsäure, Schwefelsäure, Essigsäure oder Bortrifluoridetherat, in einem Lösungsmittel wie Wasser, Alkohol, Ether, Tetrahydrofuran, Acetonitril, Dimethylformamid oder Dichlormethan durchgeführt. Die Reaktion wird zweckmäßigerweise bei Temperaturen von -10°C bis 30°C ausgeführt. Die gebildete Diazoverbindung kann als Salz, wie z.B. als Tetrafluoroborat, isoliert werden oder wird besser direkt weiter mit dem Reduktionsmittel umgesetzt. Als Reduktionsmittel eignen sich beispielsweise Hydride, wie Natriumcyanoborhydrid, Hypophosphite, Sulfite, Eisen, Eisen(II)salze oder Kupfer(I)-salze. Die Reduktion wird bevorzugt zwischen -10°C und 100°C durchgeführt.
c) Zur Herstellung einer Verbindung der allgemeinen Formel I, in der R⁴ gemäß der eingangs erwähnten Definition eine Aminogruppe oder eine gegebenenfalls im Alkylteil substituierte Alkylaminogruppe enthält:
   Entschützung einer Verbindung der allgemeinen Formel 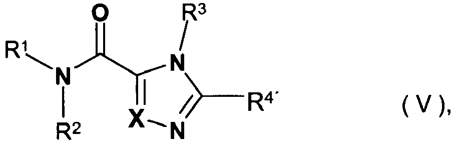
      in der R¹, R², X und R³ wie eingangs definiert sind und
      R⁴' eine N-tert.-Butyloxycarbonylamino-, N-tert.-Butyloxycarbonyl-N-alkylamino-, Phthalimido- oder Azidogruppe enthält, wobei der Alkylteil der N-tert.-Butyloxycarbonyl-N-alkylaminogruppe wie eingangs erwähnt substituiert sein kann.

Die Abspaltung eines tert.-Butyloxycarbonylrestes erfolgt vorzugsweise durch Behandlung mit einer Säure wie Trifluoressigsäure oder Salzsäure oder durch Behandlung mit Bromtrimethylsilan oder lodtrimethylsilan gegebenenfalls unter Verwendung eines Lösungsmittels wie Methylenchlorid, Essigester, Dioxan, Methanol, Isopropanol oder Diethylether bei Temperaturen zwischen 0 und 80°C.

Die Abspaltung eines Phthalylrestes erfolgt vorzugsweise in Gegenwart von Ethanolamin, Hydrazin oder eines primären Amins wie Methylamin, Ethylamin oder n-Butylamin in einem Lösungsmittel wie Methanol, Ethanol, Isopropanol, Toluol, Wasser oder Dioxan bei Temperaturen zwischen 20 und 100°C.

Die Reduktion einer Azidgruppe zum entsprechenden Amin erfolgt vorzugsweise in Gegenwart eines Phosphins wie Triphenyl-, Trimethyl-, Triethyl- oder Tributylphosphin in einem Lösungsmittel wie Tetrahydrofuran, Dioxan oder Toluol bei Temperaturen zwischen 0 und 110°C. Alternativ kann die Reduktion auch mit Wasserstoff in Gegenwart eines Übergangsmetallkatalysators wie z.B. Palladium auf Kohle in Ethylacetat, Alkohol, Ether, Essigsäure, Wasser bei Temperaturen zwischen 0 und 80°C durchgeführt werden.

Erhält man erfindungsgemäß eine Verbindung der allgemeinen Formel I, die eine Amino-, Alkylamino- oder Iminogruppe enthält, so kann diese mittels Acylierung oder Sulfonylierung in eine entsprechende Acyl- oder Sulfonylverbindung der allgemeinen Formel I übergeführt werden oder
eine Verbindung der allgemeinen Formel I, die eine Amino-, Alkylamino- oder Iminogruppe enthält, so kann diese mittels Alkylierung oder reduktiver Alkylierung in eine entsprechende Alkylverbindung der allgemeinen Formel I übergeführt werden oder
eine Verbindung der allgemeinen Formel I, die eine Carboxygruppe enthält, so kann diese mittels Veresterung in einen entsprechenden Ester der allgemeinen Formel I übergeführt werden oder
eine Verbindung der allgemeinen Formel I, die eine Carboxy- oder Estergruppe enthält, so kann diese durch Umsetzung mit einem Amin in ein entsprechendes Amid der allgemeinen Formel I übergeführt werden.

Die nachträgliche Veresterung wird gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan oder besonders vorteilhaft in einem entsprechenden Alkohol gegebenenfalls in Gegenwart einer Säure wie Salzsäure oder in Gegenwart eines wasserentziehenden Mittels, z.B. in Gegenwart von Chlorameisensäureisobutylester, Thionylchlorid, Trimethylchlorsilan, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodümid/N-Hydroxysuccinimid oder 1-Hydroxy-benztriazol und gegebenenfalls zusätzlich in Gegenwart von 4-Dimethylamino-pyridin, N,N'-Carbonyldümidazol oder Triphenylphosphin/Tetrachlorkohlenstoff, zweckmäßigerweise bei Temperaturen zwischen 0 und 150°C, vorzugsweise bei Temperaturen zwischen 0 und 80°C, durchgeführt.

Die nachträgliche Esterbildung kann auch durch Umsetzung einer Verbindung, die eine Carboxygruppe enthält, mit einem entsprechenden Alkylhalogenid erfolgen.

Die nachträgliche Acylierung oder Sulfonylierung wird gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan mit einem entsprechenden Acyl- oder Sulfonylderivat gegebenenfalls in Gegenwart einer tertiären organischen Base oder in Gegenwart einer anorganischen Base oder in Gegenwart eines wasserentziehenden Mittels, z.B. in Gegenwart von Chlorameisensäureisobutylester, Thionylchlorid, Trimethylchlorsilan, Schwefelsäure, Methansulfonsäure, p-Toluolsulfonsäure, Phosphortrichlorid, Phosphorpentoxid, N,N'-Dicyc)ohexy)carbodiimid, N,N'-Dicyclohexylcarbodiimid/N-Hydroxysuccinimid oder 1-Hydroxy-benztriazol und gegebenenfalls zusätzlich in Gegenwart von 4-Dimethylamino-pyridin, N,N'-Carbonyldiimidazol oder Triphenylphosphin/Tetrachlorkohlenstoff, zweckmäßigerweise bei Temperaturen zwischen 0 und 150°C, vorzugsweise bei Temperaturen zwischen 0 und 80°C, durchgeführt.

Die nachträgliche Alkylierung wird gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan mit einem Alkylierungsmittel wie einem entsprechenden Halogenid oder Sulfonsäureester, z.B. mit Methyljodid, Ethylbromid, Dimethylsulfat oder Benzylchlorid, gegebenenfalls in Gegenwart einer tertiären organischen Base oder in Gegenwart einer anorganischen Base zweckmäßigerweise bei Temperaturen zwischen 0 und 150°C, vorzugsweise bei Temperaturen zwischen 0 und 100°C, durchgeführt.

Die nachträgliche reduktive Alkylierung wird mit einer entsprechenden Carbonylverbindung wie Formaldehyd, Acetaldehyd, Propionaldehyd, Aceton oder Butyraldehyd in Gegenwart eines komplexen Metallhydrids wie Natriumborhydrid, Lithiumborhydrid, Natriumtriacetoxyborhydrid oder Natriumcyanoborhydrid zweckmäßigerweise bei einem pH-Wert von 6-7 und bei Raumtemperatur oder in Gegenwart eines Hydrierungskatalysators, z.B. mit Wasserstoff in Gegenwart von Palladium/Kohle, bei einem Wasserstoffdruck von 1 bis 5 bar durchgeführt. Die Methylierung kann auch in Gegenwart von Ameisensäure als Reduktionsmittel bei erhöhten Temperaturen, z.B. bei Temperaturen zwischen 60 und 120°C, durchgeführt werden.

Die nachträgliche Amidbildung wird durch Umsetzung eines entsprechenden reaktionsfähigen Carbonsäurederivates mit einem entsprechenden Amin gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan, wobei das eingesetzte Amin gleichzeitig als Lösungsmittel dienen kann, gegebenenfalls in Gegenwart einer tertiären organischen Base oder in Gegenwart einer anorganischen Base oder mit einer entsprechenden Carbonsäure in Gegenwart eines wasserentziehenden Mittels, z.B. in Gegenwart von Chlorameisensäureisobutylester, Thionylchlorid, Trimethylchlorsilan, Phosphortrichlorid, Phosphorpentoxid, N,N'-Dicyclohexylcarbodiimid, N,N'-Dicyclohexylcarbodiimid/N-Hydroxysuccinimid oder 1-Hydroxy-benztriazol und gegebenenfalls zusätzlich in Gegenwart von 4-Dimethylamino-pyridin, N,N-Carbonyldiimidazol oder Triphenylphosphin/Tetrachlorkohlenstoff, zweckmäßigerweise bei Temperaturen zwischen 0 und 150°C, vorzugsweise bei Temperaturen zwischen 0 und 80°C, durchgeführt.

Bei den vorstehend beschriebenen Umsetzungen können gegebenenfalls vorhandene reaktive Gruppen wie Hydroxy-, Carboxy-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, Methyl-, Ethyl-, tert-Butyl-, Trityl-, Benzyl- oder Tetrahydro-pyranylgruppe,
als Schutzreste für eine Carboxygruppe die Trimethylsilyl-, Methyl-, Ethyl-, tert.-Butyl-, Benzyl- oder Tetrahydropyranylgruppe,
als Schutzreste für eine Amino-, Alkylamino- oder Iminogruppe die Formyl-, Acetyl-, Trifluoracetyl-, Ethoxycarbonyl-, tert.-Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wässrigen Lösungsmittel, z.B. in Wasser, Isopropanol[Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von Jodtrimethylsilan, bei Temperaturen zwischen 0 und 120°C, vorzugsweise bei Temperaturen zwischen 10 und 100°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Raumtemperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.-Butyl- oder tert.-Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Diethylether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran bei Temperaturen zwischen 0 und 50°C.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestens 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Des Weiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxygruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Arginin, Cyclohexylamin, Ethanolamin, Diethanolamin und Triethanolamin in Betracht.

Die als Ausgangsstoffe verwendeten Verbindungen der allgemeinen Formeln II und IV sind entweder literaturbekannt oder man erhält diese nach an sich literaturbekannten Verfahren (siehe Beispiele I bis XII).

Wie bereits eingangs erwähnt, weisen die erfindungsgemäßen Verbindungen der allgemeinen Formel I und ihre physiologisch verträglichen Salze wertvolle pharmakologische Eigenschaften auf, insbesondere eine Hemmwirkung auf das Enzym DPP-IV.

Die biologischen Eigenschaften der neuen Verbindungen wurden wie folgt geprüft:
Die Fähigkeit der Substanzen und ihrer entsprechenden Salze, die DPP-IV Aktivität zu hemmen, kann in einem Versuchsaufbau gezeigt werden, in dem ein Extrakt der humanen Koloncarcinomzelllinie Caco-2 als DPP IV Quelle benutzt wird. Die Differenzierung der Zellen, um die DPP-IV Expression zu induzieren, wurde nach der Beschreibung von Reiher et al. in einem Artikel mit dem Titel "Increased expression of intestinal cell line Caco-2", erschienen in Proc. Natl. Acad. Sci. Vol. 90, Seiten 5757-5761 (1993), durchgeführt. Der Zellextrakt wurde von in einem Puffer (10mM Tris HCl, 0.15 M NaCl, 0.04 t.i.u. Aprotinin, 0.5% Nonidet-P40, pH 8.0) solubilisierten Zellen durch Zentrifugation bei 35,000 g für 30 Minuten bei 4°C (zur Entfernung von Zelltrümmern) gewonnen.
Der DPP-IV Assay wurde wie folgt durchgeführt:
50 µl Substratlösung (AFC; AFC ist Amido-4-trifluormethylcoumarin), Endkonzentration 100 µM, wurden in schwarze Mikrotiterplatten vorgelegt. 20 µl Assay Puffer (Endkonzentrationen 50 mM Tris HCl pH 7.8, 50 mM NaCl, 1 % DMSO) wurde zupipettiert. Die Reaktion wurde durch Zugabe von 30 µl solubilisiertem Caco-2 Protein (Endkonzentration 0.14 µg Protein pro Well) gestartet. Die zu überprüfenden Testsubstanzen wurden typischerweise in 20 µl vorverdünnt zugefügt, wobei das Assaypuffervolumen dann entsprechend reduziert wurde. Die Reaktion wurde bei Raumtemperatur durchgeführt, die Inkubationsdauer betrug 60 Minuten. Danach wurde die Fluoreszenz in einem Victor 1420 Multilabet Counter gemessen, wobei die Anregungswellenlänge bei 405 nm und die Emissionswellenlänge bei 535 nm lag. Leerwerte (entsprechend 0 % Aktivität) wurden in Ansätzen ohne Caco-2 Protein (Volumen ersetzt durch Assay Puffer), Kontrollwerte (entsprechend 100 % Aktivität) wurden in Ansätzen ohne Substanzzusatz erhalten. Die Wirkstärke der jeweiligen Testsubstanzen, ausgedrückt als IC₅₀ Werte, wurden aus Dosis-Wirkungs Kurven berechnet, die aus jeweils 11 Meßpunkten bestanden. Hierbei wurden folgende Ergebnisse erhalten:

| Verbindung (Beispiel Nr.) | DPP IV-Hemmung IC₅₀ [nM] |
|---|---|
| 1 | 106 |
| 1(2) | 41 |
| 1(3) | 99 |
| 2 | 367 |
| 2(2) | 386 |
| 3 | 101 |
| 3(1) | 101 |
| 4 | 382 |
| 4(1) | 24 |
| 4(2) | 36 |

Die erfindungsgemäß hergestellten Verbindungen sind gut verträglich, da beispielsweise nach oraler Gabe von 10 mg/kg der Verbindung des Beispiels 4(1) an Ratten keine Änderungen im Verhalten der Tiere beobachtet werden konnten.

Im Hinblick auf die Fähigkeit, die DPP-IV Aktivität zu hemmen, sind die erfindungsgemäßen Verbindungen der allgemeinen Formel I und ihre entsprechenden pharmazeutisch akzeptablen Salze geeignet, alle diejenigen Zustände oder Krankheiten zu beeinflussen, die durch eine Hemmung der DPP-IV Aktivität beeinflusst werden können. Es ist daher zu erwarten, daß die erfindungsgemäßen Verbindungen zur Prävention oder Behandlung von Krankheiten oder Zuständen wie Diabetes mellitus Typ 1 und Typ 2, diabetische Komplikationen (wie z.B. Retinopathie, Nephropathie oder Neuropathien), metabolische Azidose oder Ketose, reaktiver Hypoglykämie, Insulinresistenz, Metabolischem Syndrom, Dyslipidämien unterschiedlichster Genese, Arthritis, Atherosklerose und verwandte Erkrankungen, Adipositas, Allograft Transplantation und durch Calcitonin verursachte Osteoporose geeignet sind. Darüber hinaus sind diese Substanzen geeignet, die B-Zelldegeneration wie z.B. Apoptose oder Nekrose von pankreatischen B-Zellen zu verhindern. Die Substanzen sind weiter geeignet, die Funktionalität von pankreatischen Zellen zu verbessern oder wiederherzustellen, daneben die Anzahl und Größe von pankreatischen B-Zellen zu erhöhen. Zusätzlich und begründet durch die Rolle der Glucagon-Like Peptide, wie z.B. GLP-1 und GLP-2 und deren Verknüpfung mit DPP-IV Inhibition, wird erwartet, dass die erfindungsgemäßen Verbindungen geeignet sind, um unter anderem einen sedierenden oder angstlösenden Effekt zu erzielen, darüber hinaus katabole Zustände nach Operationen oder hormonelle Stressantworten günstig zu beeinflussen oder die Mortalität und Morbidität nach Myokardinfarkt reduzieren zu können. Darüber hinaus sind sie geeignet zur Behandlung von allen Zuständen, die im Zusammenhang mit oben genannten Effekten stehen und durch GLP-1 oder GLP-2 vermittelt sind. Die erfindungsgemäßen Verbindungen sind ebenfalls als Diuretika oder Antihypertensiva einsetzbar und zur Prävention und Behandlung des akuten Nierenversagens geeignet. Weiterhin sind die erfindungsgemäßen Verbindungen zur Behandlung entzündlicher Erkrankungen der Atemwege einsetzbar. Ebenso sind sie zur Prävention und Therapie von chronischen entzündlichen Darmerkrankungen wie z.B. Reizdarmsyndrom (IBS), Morbus Crohn oder Colitis ulcerosa ebenso wie bei Pankreatitis geeignet. Des Weiteren wird erwartet, dass sie bei jeglicher Art von Verletzung oder Beeinträchtigung im Gastrointestinaltrakt eingesetzt werden können wie auch z.B. bei Kolitiden und Enteriden. Darüber hinaus wird erwartet, dass DPP-IV Inhibitoren und somit auch die erfindungsgemäßen Verbindungen zur Behandlung der Unfruchtbarkeit oder zur Verbesserung der Fruchtbarkeit beim Menschen oder im Säugetierorganismus verwendet werden können, insbesondere dann, wenn die Unfruchtbarkeit im Zusammenhang mit einer Insulinresistenz oder mit dem polyzystischen Ovarialsyndrom steht. Auf der anderen Seite sind diese Substanzen geeignet, die Motilität der Spermien zu beeinflussen und sind damit als Kontrazeptiva zur Verwendung beim Mann einsetzbar. Des Weiteren sind die Substanzen geeignet, Mangelzustände von Wachstumshormon, die mit Minderwuchs einhergehen, zu beeinflussen, sowie bei allen Indikationen sinnvoll eingesetzt werden können, bei denen Wachstumshormon verwendet werden kann. Die erfindungsgemäßen Verbindungen sind auf Grund ihrer Hemmwirkung gegen DPP IV auch geeignet zur Behandlung von verschiedenen Autoimmunerkrankungen wie z.B. rheumatoide Arthritis, Multiple Sklerose, Thyreoditiden und Basedow'scher Krankheit etc.. Darüber hinaus können sie eingesetzt werden bei viralen Erkrankungen wie auch z.B. bei HIV Infektionen, zur Stimulation der Blutbildung, bei benigner Prostatahyperplasie, bei Gingivitiden sowie zur Behandlung von neuronalen Defekten und neurdegenerativen Erkrankungen wie z.B. Morbus Alzheimer. Beschriebene Verbindungen sind ebenso zu verwenden zur Therapie von Tumoren, insbesondere zur Veränderung der Tumorinvasion wie auch Metastatisierung, Beispiele hier sind die Anwendung bei T-Zell Lymphomen, akuter lymphoblastischer Leukämie, zellbasierende Schilddrüsenkarzinome, Basalzellkarzinome oder Brustkarzinome. Weitere Indikationen sind Schlaganfall, Ischämien verschiedenster Genese, Morbus Parkinson und Migräne. Darüber hinaus sind weitere Indikationsgebiete follikuläre und epidermale Hyperkeratosen, erhöhte Keratinozytenproliferation, Psoriasis, Enzephalomyelitiden, Glomerulonephritiden, Lipodystrophien sowie psychosomatische, depressive und neuropsychiatrische Erkrankungen verschiedenster Genese.

Die erfindungsgemäßen Verbindungen können auch in Kombination mit anderen Wirkstoffen verwendet werden. Zu den zu einer solchen Kombination geeigneten Therapeutika gehören z.B. Antidiabetika, wie etwa Metformin, Sulfonylharnstoffe (z.B. Glibenclamid, Tolbutamid, Glimepiride), Nateglinide, Repaglinide, Thiazolidindione (z.B. Rosiglitazone, Pioglitazone), PPAR-gamma-Agonisten (z.B. Gl 262570) und -Antagonisten, PPAR-gamma/alpha Modulatoren (z.B. KRP 297), alpha-Glucosidasehemmer (z.B. Acarbose, Voglibose),andere DPPIV Inhibitoren, alpha2-Antagonisten, Insulin und Insulinanaloga, GLP-1 und GLP-1 Analoga (z.B. Exendin-4) oder Amylin. Daneben SGLT2-Inhibitoren wie T-1095 oder KGT-1251 (869682), Inhibitoren der Proteintyrosinphosphatase 1, Substanzen, die eine deregulierte Glucoseproduktion in der Leber beeinflussen, wie z.B. Inhibitoren der Glucose-6-phosphatase, oder der Fructose-1,6-bisphosphatase, der Glycogenphosphorylase, Glucagonrezeptor Antagonisten und Inhibitoren der Phosphoenolpyruvatcarboxykinase, der Glykogensynthasekinase oder der Pyruvatdehydrokinase, Lipidsenker, wie etwa HMG-CoA-Reduktasehemmer (z.B. Simvastatin, Atorvastatin), Fibrate (z.B. Bezafibrat, Fenofibrat), Nikotinsäure und deren Derivate, PPAR-alpha agonisten, PPAR-delta agonisten, ACAT Inhibitoren (z.B. Avasimibe) oder Cholesterolresorptionsinhibitoren wie zum Beispiel Ezetimibe, gallensäurebindende Substanzen wie zum Beispiel Colestyramin, Hemmstoffe des ilealen Gallensäuretransportes, HDLerhöhende Verbindungen wie zum Beispiel Inhibitoren von CETP oder Regulatoren von ABC1 oder Wirkstoffe zur Behandlung von Obesitas, wie etwa Sibutramin oder Tetrahydrolipstatin, Dexfenfluramin, Axokine, Antagonisten des Cannbinoid1 Rezeptors, MCH-1 Rezeptorantagonisten, MC4 Rezeptor Agonisten, NPY5 oder NPY2 Antagonisten oder ß₃-Agonisten wie SB-418790 oder AD-9677 ebenso wie Agonisten des 5HT2c Rezeptors.

Daneben ist eine Kombination mit Medikamenten zur Beeinflussung des Bluthochdrucks wie z.B. All Antagonisten oder ACE Inhibitoren, Diuretika, ß-Blocker, Ca-Antagonisten und anderen oder Kombinationen daraus geeignet.

Die zur Erzielung einer entsprechenden Wirkung erforderliche Dosierung beträgt zweckmäßigerweise bei intravenöser Gabe 1 bis 100 mg, vorzugsweise 1 bis 30 mg, und bei oraler Gabe 1 bis 1000 mg, vorzugsweise 1 bis 100 mg, jeweils 1 bis 4 x täglich. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/ Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen oder Zäpfchen einarbeiten.

Die nachfolgenden Beispiele sollen die Erfindung näher erläutern:

### Herstellung der Ausgangsverbindungen:

### Beispiel I

### 1-Ethoxycarbonylmethyl-3-cyan-2-phenyl-isoharnstoff

Zu einer Lösung von 50,0 g Diphenyl-N-cyan-carbonimidat in 29 ml Triethylamin und 500 ml Isopropanol werden 29,3 g Glycinethylesterhydrochlorid gegeben. Die Lösung wird 16 h (Stunden) bei Raumtemperatur gerührt und dann eingeengt. Der Rückstand wird in Ethylacetat gelöst und die organische Phase mit Wasser und wässriger Kaliumcarbonatlösung gewaschen. Die organische Phase wird über Natriumsulfat getrocknet und das Lösungsmittel vollständig entfernt. Der Rückstand wird mit Diethylether gewaschen und getrocknet.
Ausbeute: 35,5 g (68% der Theorie)
Massenspektrum (ESI⁺): m/z = 248 [M+H]⁺

### Beispiel II

### 1-Ethoxycarbonylmethy-1-(but-2-inyl)-3-cyan-2-phenyl-isoharnstoff

Zu einem Gemisch von 30,2 g 1-Ethoxycarbonylmethyl-3-cyan-2-phenyl-isoharnstoff und 20,0 g Kaliumcarbonat in 200 ml Aceton werden 11 ml But-2-inylbromid gegeben. Nach 1 d (Tag) Rühren bei Raumtemperatur werden noch einmal 6,5 g Kaliumcarbonat und 3,5 ml But-2-inylbromid zugegeben. Nach weiteren 20 h bei Raumtemperatur wird das Lösungsmittel entfernt und Ethylacetat zugegeben. Die organische Phase wird mit Wasser gewaschen, über Natriumsulfat getrocknet und bis zur Trockene eingeengt.
Ausbeute: 35,2 g (96% der Theorie)
Massenspektrum (ESI⁺): m/z = 300 [M+H]⁺

### Beispiel III

### 3-tert-Butoxycarbonylamino-N-(ethoxycarbonylmethyl)-N-(but-2-inyl)-N'-cyan-piperidin-1-carboxamidin

Zu einem Gemisch von 10,0 g 3-tert-Butoxycarbonylaminopiperidin und 4,8 g Kaliumcarbonat in 50 ml Dimethylformamid werden 10,0 g 1-Ethoxycarbonylmethyl-1-(but-2-inyl)-3-cyan-2-phenyl-isoharnstoff gegeben. Das Reaktionsgemisch wird 1 d bei Raumtemperatur gerührt und danach werden noch einmal 1,6 g Kaliumcarbonat und 3,0 g 3-tert-Butoxycarbonylaminopiperidin zugegeben. Nach weiteren 3 d bei Raumtemperatur wird Wasser zugegeben und mit Ethylacetat extrahiert. Die organischen Extrakte werden über Natriumsulfat getrocknet, das Lösungsmittel wird entfernt und der Rückstand über Kieselgel gereinigt (Cyclohexan/Ethylacetat 5:1->1:2).
Ausbeute: 12,5 g (ca. 90%ig, 83% der Theorie)
Massenspektrum (ESI⁺): m/z = 406 [M+H]⁺

### Beispiel IV

### 5-Amino-2-(3-tert-butoxycarbonylamino-piperidin-1-yl)-3-(but-2-inyl)-3H-imidazol-4-carbonsäureethylester

Zu einer Lösung von 12,5 g (ca. 90%ig) 3-tert-Butoxycarbonylamino-N-(ethoxy-carbonylmethyl)-N-(but-2-inyl)-N'-cyan-piperidin-1-carboxamidin in 100 ml trockenem Ethanol werden 2,5 g Natriumethanolat gegeben. Die Reaktionslösung wird 3 h bei Raumtemperatur gerührt und dann mit 1 M Salzsäure neutralisiert. Das Lösungsmittel wird entfernt, Wasser zugegeben und mit Ethylacetat extrahiert. Die organischen Extrakte werden über Natriumsulfat getrocknet, das Lösungsmittel wird entfernt und der Rückstand über Kiesel gereinigt (Cyclohexan/Ethylacetat 3:1->1:5).
Ausbeute: 5,7 g (51 % der Theorie)
Massenspektrum (ESI⁺): m/z = 406 [M+H]⁺

### Beispiel V

### 2-(3-tert-Butoxycarbonylamino-piperidin-1-yl)-3-(but-2-inyl)-3H-imidazol-4-carbonsäureethlyester

Eine Lösung von 3,00 g 5-Amino-2-(3-tert-butoxycarbonylamino-piperidin-1-yl)-3-(but-2-inyl)-3H-imidazol-4-carbonsäureethylester in 36 ml 2 M Salzsäure wird auf 0°C abgekühlt. Dazu gibt man eine Lösung von 0,60 g Natriumnitrit in 2 ml Wasser und rührt das Reaktionsgemisch 1 h bei 0°C. Dann werden 8,4 ml Hypophosphorige Säure zugegeben und weitere 14 h bei 0°C gerührt. Danach gibt man das Reaktionsgemisch zu eisgekühlter wässriger Kaliumcarbonatlösung und extrahiert mit Dichlormethan. Die organischen Extrakte werden über Natriumsulfat getrocknet und eingeengt. Der Rückstand wird in 8 ml Dichlormethan gelöst, und 1,62 g Kohlensäure-di-tert-butylester werden zugegeben. Die Lösung wird 2 h bei Raumtemperatur gerührt, dann bis zur Trockene eingeengt und über Kieselgel chromatografiert (Cyclohexan/Ethylacetat 7:3->1:1).
Ausbeute: 1,62 g (56% der Theorie)
Massenspektrum (ESI⁺): m/z = 391 [M+H]⁺

### Beispiel VI

### 2-(3-tert-Butoxycarbonylamino-piperidin-1-yl)-3-(but-2-inyl)-3H-imidazol-4-carbonsäure

Eine Lösung von 4,40 g 2-(3-tert-Butoxycarbonylamino-piperidin-1-yl)-3-(but-2-inyl)-3H-imidazol-4-carbonsäureethylester in 28 ml 4 M Kaliumhydroxid-Lösung und 20 ml Tetrahydrofuran wird 14 h bei 100°C gerührt. Danach wird die Lösung mit 2 M Salzsäure neutralisiert und mit Dichlormethan extrahiert. Die vereinten organischen Extrakte werden über Natriumsulfat getrocknet und bis zur Trockene eingeengt.
Ausbeute: 3,12 g (76% der Theorie)
Massenspektrum (ESI⁺): m/z = 363 [M+H]⁺

### Beispiel VII

### 1-(but-2-inyl)-2-(3-tert-butoxycarbonylamino-piperidin-1-yl)-5-(naphth-1-ylmethylaminocarbonyl)-1H-imidazol

Zu einer Lösung von 0,20 g 2-(3-tert-Butoxycarbonylamino-piperidin-1-yl)-3-(but-2-inyl)-3H-imidazol-4-carbonsäure und 0,19 g O-(Benzotriazol-1-yl)-N,N,N',N'-tetramethyluroniumtetrafluoroborat in 5 ml Dimethylformamid werden 0,09 ml 1-Amino-methylnaphthalin und 0,28 ml Ethyldiisopropylamin gegeben. Die Lösung wird 3 h bei Raumtemperatur gerührt und dann mit gesättigter wässriger Kaliumcarbonatlösung versetzt. Danach wird mit Ethylacetat extrahiert, die organischen Extrakte werden über Natriumsulfat getrocknet, und das Lösungsmittel wird entfernt. Der Rückstand wird über Kieselgel gereinigt (Dichlormethan/Methanol 1:0->7:3).
Ausbeute: 0,18 g (65% der Theorie)
Massenspektrum (ESI⁺): m/z = 502 [M+H]⁺

Analog Beispiel VII werden folgende Verbindungen erhalten:
(1) 1-(But-2-inyl)-2-(3-tert-butoxycarbonylamino-piperidin-1-yl)-5-[N-(naphth-1-ylmethyl)-N-methyl-aminocarbonyl]-1H-imidazol
   Massenspektrum (ESI: m/z = 516 [M+H]⁺
(2) 1-(But-2-inyl)-2-(3-tert-butoxycarbonylamino-piperidin-1-yl)-5-(phenanthridin-6-ylmethylaminocarbonyl)-1H-imidazol
   Massenspektrum (ESI⁺): m/z = 553 [M+H]⁺
(3) 1-(But-2-inyl)-2-(3-tert-butoxycarbonylamino-piperidin-1-yl)-5-(3-methyl-isochinolin-1-ylmethylaminocarbonyl)-1 H-imidazol
   Massenspektrum (ESI⁺): m/z = 517 [M+H]⁺
(4) 1-(But-2-inyl)-2-(3-tert-butoxycarbonylamino-piperidin-1-yl)-5-(chinolin-2-yl-methylaminocarbonyl)-1 H-imidazol
   Massenspektrum (ESI⁺): m/z = 503 [M+H]⁺
(5) 1-(But-2-inyl)-2-(3-tert-butoxycarbonylamino-piperidin-1-yl)-5-(chinolin-3- yl-methylaminocarbonyl)-1 H-imidazol
   Massenspektrum (ESI⁺): m/z = 503 [M+H]⁺
(6) 1-(But-2-inyl)-2-(3-tert-butoxycarbonylamino-piperidin-1-yl)-5-(2-phenyl-ethyl-aminocarbonyl)-1 H-imidazol
   Massenspektrum (ESI⁺): m/z = 466 [M+H]⁺

### Beispiel VIII

### 2-Hydrazino-N-(naphth-1 -ylmethyl)-2-oxo-acetamid

Zu einer Lösung von 1,0 g Hydrazino-oxo-essigsäuremethylester in 10 ml Methanol werden 1,25 ml 1-Aminomethylnaphthalin gegeben. Die Lösung wird 16 h bei Raumtemperatur gerührt und danach teilweise vom Methanol befreit. Der Niederschlag wird abgetrennt, mit Diethylether gewaschen und getrocknet.
Ausbeute: 1,77 g (86% der Theorie)
Massenspektrum (ESI⁺): m/z = 244 [M+H]⁺

Analog Beispiel VIII werden folgende Verbindungen erhalten:
(1) 2-Hydrazino-N-(3-methyl-isochinolin-1-ylmethyl)-2-oxo-acetamid Massenspektrum (ESI⁺): m/z = 259 [M+H]⁺
   Die Reaktionslösung wird auf 60°C erwärmt.
(2) 2-Hydrazino-2-oxo-N-(chinolin-6-ylmethyl)-acetamid
   Massenspektrum (ESI⁺): m/z = 245 [M+H]⁺
(3) 2-Hydrazino-2-oxo-N-(phenanthridin-6-ylmethyl)-acetamid
   Massenspektrum (ESI⁺): m/z = 295 [M+H]⁺
Die Reaktion wird in Dimethylformamid in Gegenwart von Kaliumcarbonat bei 40°C durchgeführt.

### Beispiel IX

### 1-(Naphth-1-ylmethylaminooxalyl)-4-benzyl-thiosemicarbazid

Zu einer Lösung von 0,5 g 2-Hydrazino-N-naphth-1-ylmethyl-2-oxo-acetamid in 10 ml Dioxan werden 0,28 ml Benzylisothiocyanat gegeben. Danach wird das Lösungsmittel vollständig entfernt.
Ausbeute: 0,84 g (99% der Theorie)
Massenspektrum (ESI⁺): m/z = 393 [M+H]⁺

Analog Beispiel IX werden folgende Verbindungen erhalten:
(1) 1-(Naphth-1-ylmethylaminooxalyl)-4-(3-methyl-but-2-enyl)-thiosemicarbazid Massenspektrum (ESI⁺): m/z = 371 [M+H]⁺
(2) 1-((3-Methyl-isochinolin-1-yl)methylaminooxalyl]-4-(but-2-inyl)-thiosemicarbazid Massenspektrum (ESI⁺): m/z = 370 [M+H]⁺
(3) 1-(Naphth-1-ylmethylaminooxalyl)-4-(2-chlor-benzyl)-thiosemicarbazid Massenspektrum (ESI⁺): m/z = 427/429 (Chlor) [M+H]⁺
(4) 1-(3-Chinolin-6-ylmethylaminooxalyl)-4-(but-2-inyl)-thiosemicarbazid Massenspektrum (ESI⁺): m/z = 356 [M+H]⁺
(5) 1-(Phenanthridin-6-ylmethylaminooxalyl]-4-(but-2-inyl)-thiosemicarbazid Massenspektrum (ESI⁺): m/z = 406 [M+H]⁺

### Beispiel X

### 5-Benzylamino-[1,3,4]oxadiazol-2-carbonsäure-(naphth-1-ylmethyl)-amid

Eine Lösung von 0,2 g 1-(Naphth-1-ylmethylaminooxalyl)-4-benzyl-thiosemicarbazid in 5 ml Dichlormethan wird im Eisbad abgekühlt. Dann werden 45 µl Methylsulfonylchlorid und 0,11 ml Triethylamin zugegeben. Die Lösung wird 3 h bei Raumtemperatur gerührt und dann mit 30 ml Dichlormethan verdünnt. Nach Waschen mit wässriger Natriumhydrogencarbonatlösung wird über Natriumsulfat getrocknet. Nach Entfernen des Lösungsmittels wird das Rohprodukt erhalten, das direkt weiter mit Piperazin umgesetzt wird (siehe Beispiel 2(1)).

Analog Beispiel X werden folgende Verbindungen erhalten:
(1) 5-(3-Methyl-but-2-enyl)amino-[1,3,4]oxadiazol-2-carbonsäure-(naphth-1-yl-methyl)-amid
   Massenspektrum (ESI⁺): m/z = 337 [M+H]⁺
(2) 5-(But-2-inyl)amino-[1,3,4]oxadiazol-2-carbonsäure-(3-methylisochinolin-1-yl-methyl)-amid
   Massenspektrum (ESI⁺): m/z = 336 [M+H]⁺
(3) 5-(2-Chlorbenzyl)amino-[1,3,4]oxadiazol-2-carbonsäure-(naphth-1-ylmethyl)-amid
   Massenspektrum (ESI⁺): m/z = 393/395 (Chlor) [M+H]⁺
(4) 5-(But-2-inyl)amino-[1,3,4]oxadiazol-2-carbonsäure-(chinolin-6-ylmethyl)-amid
   Massenspektrum (ESI⁺): m/z = 322 [M+H]⁺
(5) 5-(But-2-inyl)amino-[1,3,4]oxadiazol-2-carbonsäure-(phenanthridin-6-ylmethyl)-amid
   Massenspektrum (ESI⁺): m/z = 372 [M+H]⁺

### Beispiel XI

### 3-(3-Hydroxy-piperidin-1-yl)-4-(3-methyl-but-2-enyl)-5-(naphth-1-ylmethylamino-carbonyl)-4H-[1,2,4]triazol

Eine Mischung von 0,12 g 5-(3-Methyl-but-2-enyl)amino-[1,3,4]oxadiazol-2-carbonsäure-(naphth-1-ylmethyl)-amid und 1,80 g 3-Hydroxypiperidin wird 20 min bei 200°C in einer Mikrowelle gerührt. Nach Abkühlen auf Raumtemperatur wird Dichlormethan zugegeben, die organische Phase jeweils einmal mit Wasser und 1 M Salzsäure gewaschen, über Natriumsulfat getrocknet und das Lösungsmittel entfernt.
Ausbeute: 39 mg (26% der Theorie)
Massenspektrum (ESI⁺): m/z = 420 [M+H]⁺

Analog Beispiel XI werden folgende Verbindungen erhalten:
(1) 3-(3-Hydroxy-piperidin-1-yl)-4-benzyl-5-(naphth-1-ylmethylaminocarbonyl)-4H-[1,2,4]triazol
   Massenspektrum (ESI⁺): m/z = 442 [M+H]⁺
(2) 3-(3-Hydroxy-piperidin-1-yl)-4-(but-2-inyl)-5-(3-methyl-isochinolin-1-ylmethyl-aminocarbonyl)-4H-[1,2,4]triazol
   Massenspektrum (ESI⁺): m/z = 419 [M+H]⁺
(3) 3-(3-Hydroxy-piperidin-1-yl)-4-(2-chlor-benzyl)-5-(naphth-1-ylmethylamino-carbonyl)-4H-[1,2,4]triazol
   Massenspektrum (ESI⁺): m/z = 476/478 (Chlor) [M+H]⁺
(4) 3-(3-Hydroxy-piperidin-1-yl)-4-(but-2-inyl)-5-(chinolin-6-ylmethylaminocarbonyl)-4H-[1,2,4]triazol
   Massenspektrum (ESI⁺): m/z = 405 [M+H]⁺
(5) 3-(3-Ethoxycarbonyl-piperidin-1-yl)-4-(but-2-inyl)-5-(phenanthridin-6-ylmethyl-aminocarbonyl)-4H-[1,2,4]triazol
Die Reaktion wird analog durchgeführt, nur statt 3-Hydroxypiperidin wird 3-Ethoxy-carbonylpiperidin eingesetzt. Das Produkt wird dann direkt weiter umgesetzt (siehe Beispiel 5)

### Beispiel XII

### 3-(3-Methylsulfonyloxy-piperidin-1-yl)-4-(2-chlor-benzyl)-5-(naphth-1-ylmethylamino-carbonyl)-4H-[1,2,4]triazol

Zu einer eisgekühlten Suspension von 0,21 g 3-(3-Hydroxy-piperidin-1-yl)-4-(2-chlorbenzyl)-5-(naphth-1-ylmethylaminocarbonyl)-4H-[1,2,4]triazol in 4 ml Dichlormethan werden 0,11 ml Triethylamin und 40 µl Methylsulfonylchlorid gegeben. Das Reaktionsgemisch wird 1 h im Eisbad und dann 1 h bei Raumtemperatur gerührt. Danach wird mit Dichlormethan verdünnt und die organische Phase zwei Mal mit 1 M Salzsäure und einmal mit Wasser gewaschen. Nach Trocknen über Natriumsulfat wird das Lösungsmittel vollständig entfernt.
Ausbeute: 0,24 g (99% der Theorie)
Massenspektrum (ESI⁺): m/z = 554/556 (Chlor) [M+H]⁺

Analog Beispiel XII werden folgende Verbindungen erhalten:
(1) 3-(3-Methylsulfonyloxy-piperidin-1-yl)-4-(but-2-inyl)-5-(3-methyl-isochinolin-1-ylmethylaminocarbonyl)-4H-[1,2,4]triazol
   Massenspektrum (ESI⁺): m/z = 497 [M+H]⁺
(2) 3-(3-Methylsulfonyloxy-piperidin-1-yl)-4-(but-2-inyl)-3-(chinolin-6-ylmethylamino-carbonyl)-4H-[1,2,4]triazol
   Massenspektrum (ESI⁺): m/z = 483 [M+H]⁺

### Herstellung der Endverbindungen:

### Beispiel 1

### 1-(But-2-inyl)-2-(3-amino-piperidin-1-yl)-5-(naphth-1-ylmethylaminocarbonyl)-1H-imidazol

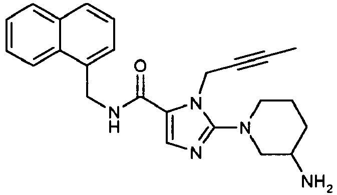

Zu einer Lösung von 0,18 g 1-(But-2-inyl)-2-(3-tert-butoxycarbonylamino-piperidin-1-yl)-5-(naphthyl-1-ylmethylaminocarbonyl)-1 H-imidazol in 4 ml Dichlormethan werden 0,62 ml Trifluoressigsäure gegeben. Nach 3 h Rühren bei Raumtemperatur wird die Reaktionslösung zu wässriger gesättigter Kaliumcarbonatlösung gegeben. Die wässrige Phase wird mit Dichlormethan extrahiert, die vereinten organischen Phasen werden über Natriumsulfat getrocknet, und das Lösungsmittel wird entfernt. Der Rückstand wird über Kieselgel chromatografiert (Dichlormethan/Methanol 1:0->7:3).
Ausbeute: 0,15 g (79% der Theorie)
Massenspektrum (ESI⁺): m/z = 402 [M+H]⁺

Analog Beispiel 1 werden folgende Verbindungen erhalten:
(1) 1-(But-2-inyl)-2-(3-amino-piperidin-1-yl)-5-[N-(naphth-1-ylmethyl)-N-methyl-aminocarbonyl]-1 H-imidazol
   Massenspektrum (ESI⁺): m/z = 416 [M+H]⁺ 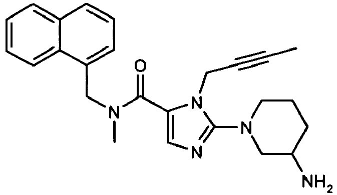
(2) 1-(But-2-inyl)-2-(3-amino-piperidin-1-yl)-5-(phenanthridin-6-ylmethylamino-carbonyl)-1 H-imidazol als Trifluoressigsäuresalz
   Massenspektrum (ESI⁺): m/z = 453 [M+H]⁺ 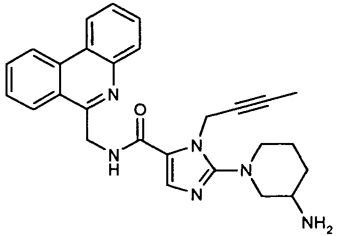
(3) 1-(But-2-inyl)-2-(3-amino-piperidin-1-yl)-5-(3-methyl-isochinolin-1-ylmethylamino-carbonyl)-1 H-imidazol
   Massenspektrum (ESI⁺): m/z = 417 [M+H]⁺ 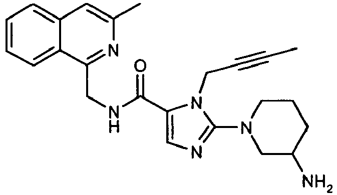
(4) 1-(But-2-inyl)-2-(3-amino-piperidin-1-yl)-5-(chinolin-3-ylmethylaminocarbonyl)-1 H-imidazol als Trifluoressigsäuresalz
   Massenspektrum (ESI⁺): m/z = 403 [M+H]⁺ 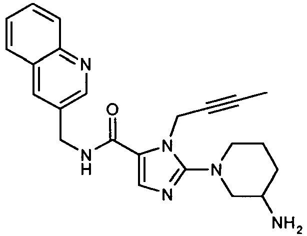
(5) 1-(But-2-inyl)-2-(3-amino-piperidin-1-yl)-5-(2-phenyl-ethylaminocarbonyl)-1 H-imidazol als Trifluoressigsäuresalz
   Massenspektrum (ESI⁺): m/z = 366 [M+H]⁺

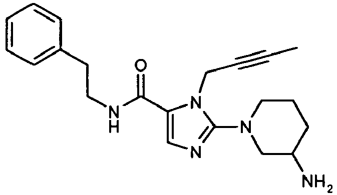

### Beispiel 2

### 3-(Piperazin-1-yl)-4-(3-methyl-but-2-enyl)-5-(naphth-1-ylmethylaminocarbonyl)-4H-[1,2,4]triazol

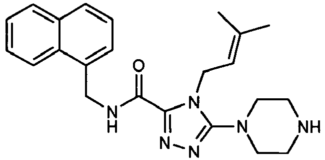

Eine Mischung von 0,10 g 5-(3-Methyl-but-2-en-1-ylamino)-[1,3,4]oxadiazol-2-carbonsäure-(naphth-1-ylmethyl)-amid und 1,28 g Piperazin wird 15 min bei 200°C in einer Mikrowelle gerührt. Nach Abkühlen auf Raumtemperatur wird Dichlormethan zugegeben, die organische Phase mit Wasser gewaschen, über Natriumsulfat getrocknet und das Lösungsmittel entfernt. Der Rückstand wird über Kieselgel chromatografisch gereinigt (Dichlormethan/Methanol 1:0->7:3).
Ausbeute: 85 mg (71 % der Theorie)
Massenspektrum (ESI⁺): m/z = 405 [M+H]⁺

Analog Beispiel 2 werden folgende Verbindungen erhalten:
(1) 3-(Piperazin-1-yl)-4-benzyl-5-(naphth-1-ylmethylaminocarbonyl)-4H-[1,2,4]triazol Massenspektrum (ESI⁺): m/z = 427 [M+H]⁺ 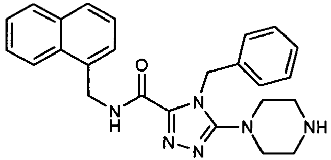
(2) 3-([1,4]Diazepan-1-yl)-4-(3-methyl-but-2-enyl)-5-(naphth-1-ylmethylamino-carbonyl)-4H-[1,2,4]triazol
   Massenspektrum (ESI⁺): m/z = 419 [M+H]⁺

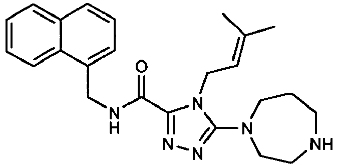

### Beispiel 3

### 3-(3-Amino-piperidin-1-yl)-4-(3-methyl-but-2-enyl)-5-(naphth-1-ylmethylamino-carbonyl)-4H-[1,2,4]triazol

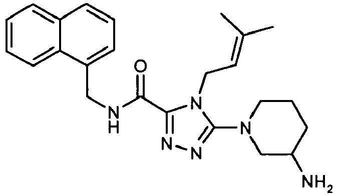

Zu einer Lösung von 39 mg 3-(3-Hydroxy-piperidin-1-yl)-4-(3-methyl-but-2-enyl)-5-(naphth-1-ylmethylaminocarbonyl)-4H-[1,2,4]triazol in 1 ml trockenem Tetrahydrofuran werden 14 mg Phthalimid, 65 mg Triphenylphosphin und zuletzt 50 µl Diisopropylazodicarboxylat gegeben. Die Lösung wird 14 h bei Raumtemperatur gerührt und dann bis zur Trockene eingeengt. Der Rückstand wird in 2 ml Toluol aufgenommen und mit 50 µl Ethanolamin versetzt. Nach 4 h Rühren bei 80°C wird auf Raumtemperatur abgekühlt und das Lösungsmittel entfernt. Der Rückstand wird über Kieselgel chromatografisch gereinigt (Dichlormethan/Methanol/Ammoniumhydroxid 95:5:1->80:20:1).
Ausbeute: 3 mg (8% der Theorie)
Massenspektrum (ESI⁺): m/z = 419 [M+H]⁺

Analog Beispiel 3 wird folgende Verbindung erhalten:
(1) 3-(3-Amino-piperidin-1-yl)-4-benzyl-5-(naphth-1-ylmethylaminocarbonyl)-4H-[1,2,4]triazol
   Massenspektrum (ESI⁺): m/z = 441 [M+H]⁺

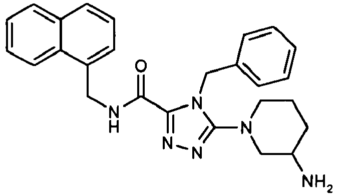

### Beispiel 4

### 3-(3-Amino-Piperidin-1-yl)-4-(2-chlor-benzyl)-5-(naphth-1-ylmethylaminocarbonyl)-4H-[1,2,4]triazol

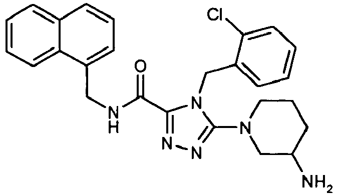

Zu einer Lösung von 0,25 g 3-(3-Hydroxy-piperidin-1-yl)-4-(2-chlor-benzyl)-5-(naphth-1-ylmethylaminocarbonyl)-4H-[1,2,4]triazol in 3 ml Dimethylformamid werden 0,09 g Natriumazid gegeben. Das Reaktionsgemisch wird 14 h bei 110°C gerührt. Nach Abkühlen auf Raumtemperatur wird Dichlormethan zugegeben und mit Wasser gewaschen. Dir organische Phase wird über Natriumsulfat getrocknet und danach das Dichlormethan entfernt. Der Rückstand wird in 4 ml Tetrahydrofuran gelöst und mit 0,7 ml 1 M Trimethylphosphinlösung in Tetrahydrofuran versetzt. Das Reaktionsgemisch wird 16 h bei Raumtemperatur gerührt und dann mit Dichlormethan verdünnt. Dir organische Phase wird mit Wasser und wässriger gesättigter Natriumhydrogencarbonatlösung gewaschen, über Natriumsulfat getrocknet und vom Lösungsmittel befreit. Der Rückstand wird über Kieselgel chromatografisch gereinigt (Dichlormethan/Methanol/Ammoniumhydroxid 95:5:1->80:20:1).
Ausbeute: 77 mg (36% der Theorie)
Massenspektrum (ESI⁺): m/z = 475/477 (Chlor) [M+H]⁺

Analog Beispiel 4 werden folgende Verbindungen erhalten:
(1) 3-(3-Amino-piperidin-1-yl)-4-(but-2-inyl)-5-(3-methyl-isochinolin-1-ylmethylamino-carbonyl)-4H-[1,2,4]triazol
   Massenspektrum (ESI⁺): m/z = 418 [M+H]⁺ 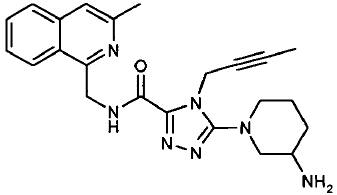
(2) 3-(3-Amino-piperidin-1-yl)-4-(but-2-inyl)-5-(chinolin-6-ylmethylaminocarbonyl)-4H-[1,2,4]triazol
   Massenspektrum (ESI⁺): m/z = 404 [M+H]⁺

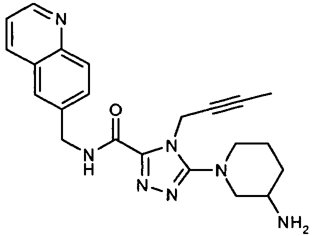

### Beispiel 5

### 3-(3-Amino-piperidin-1-yl)-4but-2-inyl)-5-(phenanthridin-6-ylmethylaminocarbonyl)-4H-[1,2,4]triazol

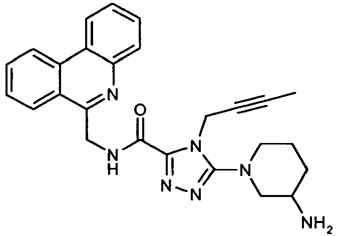

Eine Lösung von 0,15 g 3-(3-Ethoxycarbonyl-piperidin-1-yl)-4-(but-2-inyl)-5-(phenan-thridin-6-ylmethylaminocarbonyl)-4H-[1,2,4]triazol in 0,5 ml Natronlauge und 1 ml Tetrahydrofuran wird 2 h bei 45°C gerührt. Danach wird die Lösung mit 2 M Salzsäure sauer gestellt und mit Ethylacetat extrahiert. Die organischen Extrakte werden über Natriumsulfat getrocknet und das Lösungsmittel vollständig entfernt. Der Rückstand wird in 3 ml 1,2-Dichlorethan gelöst. Zur Lösung werden 0,12 ml Triethylamin und 0,08 ml Diphenylphosporylazid gegeben. Danach wird die Lösung 2 h bei 80°C gerührt. Nach Abkühlen auf Raumtemperatur wird mit Dichlormethan verdünnt und jeweils einmal mit 1 M Natronlauge und wässriger Natriumhydrogencarbonatlösung gewaschen. Die organische Phase wird über Natriumsulfat getrocknet und das Lösungsmittel entfernt. Der Rückstand wird in 1 ml Tetrahydrofuran gelöst und mit 1 ml 1 M Natronlauge behandelt. Nach 1 h Rühren wird wässrige Natriumhydrogencarbonatlösung zugeben, mit Dichlormethan extrahiert und über Natriumsulfat getrocknet. Der Rückstand wird über Kieselgel chromatografisch gereinigt (Dichlormethan/Methanol/Ammoniumhydroxid 98:2:1->80:20:1).
Ausbeute: 22 mg (16% der Theorie)
Massenspektrum (ESI⁺): m/z = 454 [M+H]⁺

Analog den vorstehenden Beispielen und anderen literaturbekannten Verfahren können auch die folgenden Verbindungen erhalten werden:

| **Bsp.** | **Struktur** | **Bsp.** | **Struktur** |
|---|---|---|---|
| **(1)** | 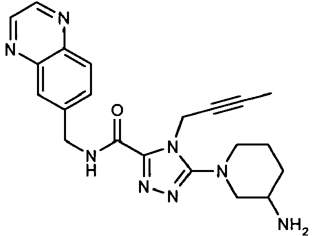 | **(2)** | 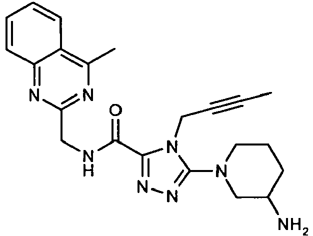 |
| **(3)** | 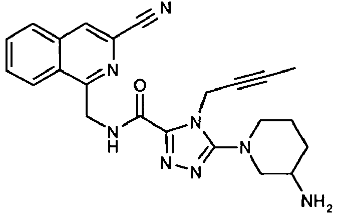 | **(4)** | 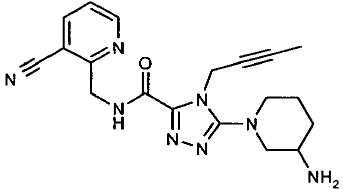 |
| **(5)** | 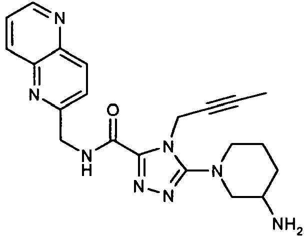 | **(6)** | 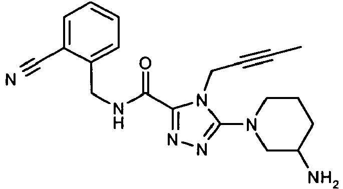 |
| **(7)** | 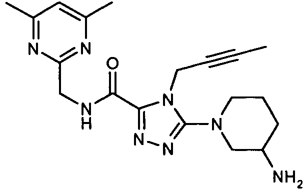 | **(8)** | 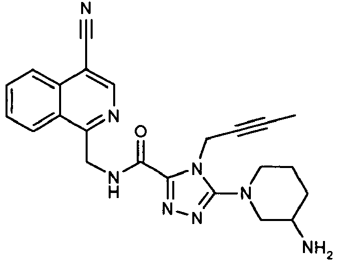 |
| **(9)** | 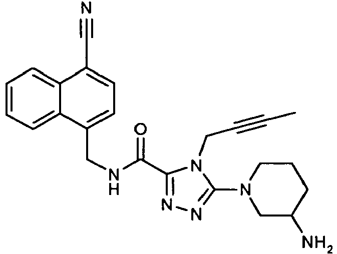 | **(10)** | 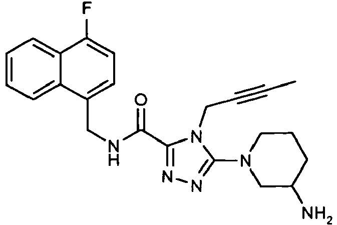 |
| **(11)** | 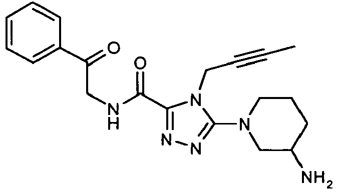 | **(12)** | 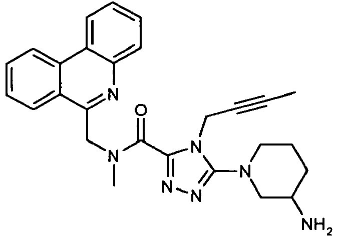 |
| **(13)** | 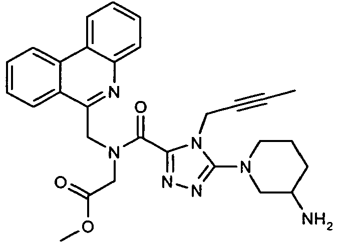 | **(14)** | 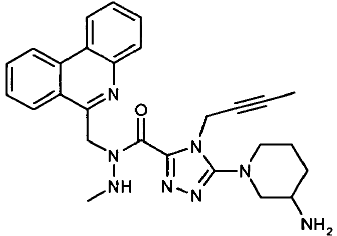 |
| **(15)** | 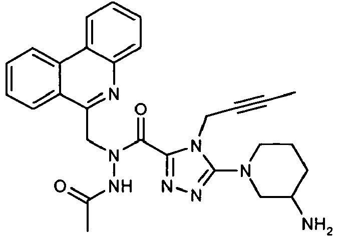 | **(16)** | 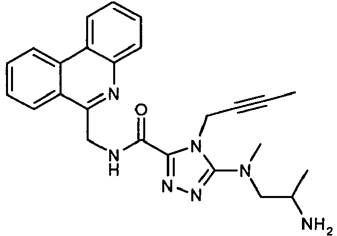 |
| **(17)** | 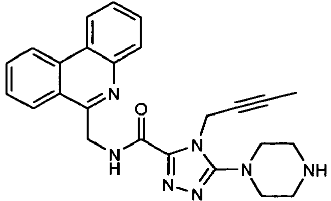 | **(18)** | 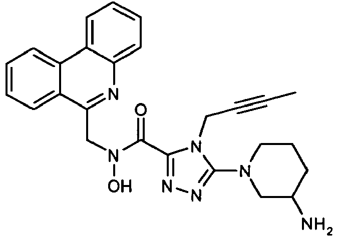 |
| **(19)** | 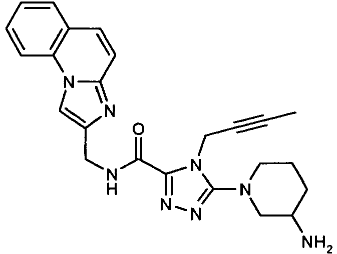 | **(20)** | 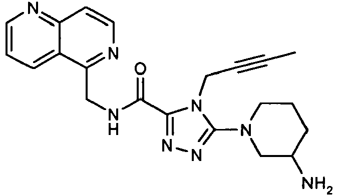 |
| **(21)** | 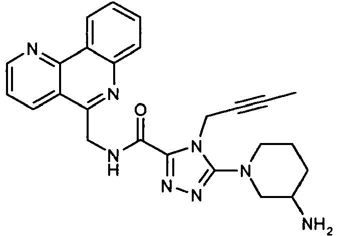 | **(22)** | 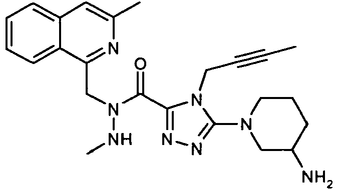 |
| **(23)** | 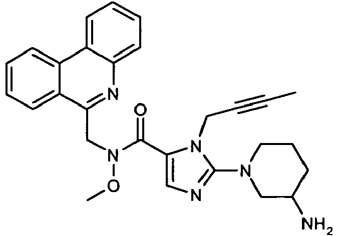 | **(24)** | 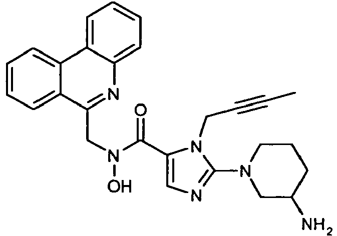 |
| **(25)** | 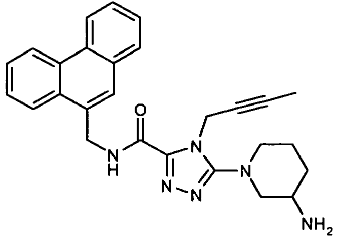 | **(26)** | 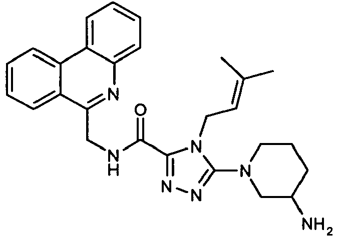 |
| **(27)** | 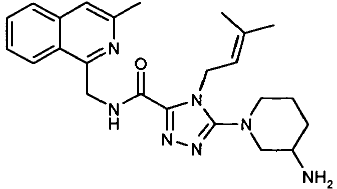 | **(28)** | 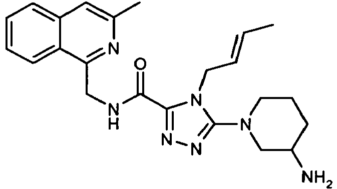 |
| **(29)** | 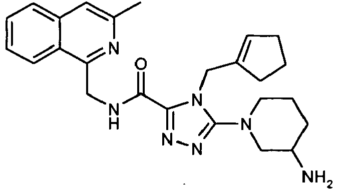 | **(30)** | 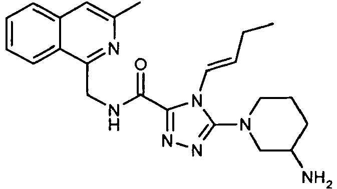 |
| **(31)** | 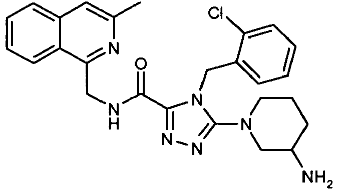 | **(32)** | 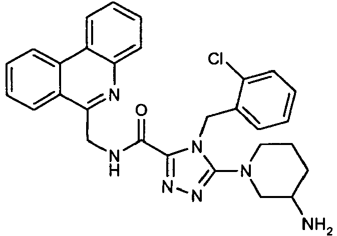 |
| **(33)** | 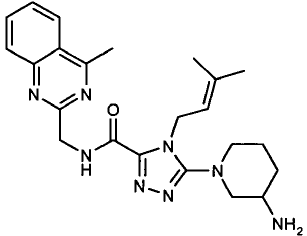 | **(34)** | 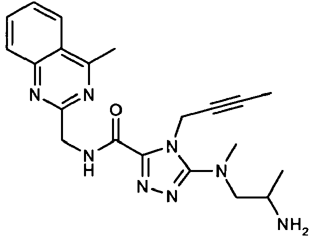 |
| **(35)** | 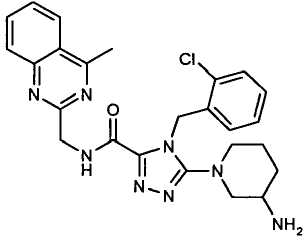 | **(36)** | 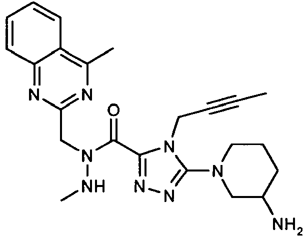 |

### Beispiel 6

### Dragees mit 75 mg Wirksubstanz

### 1 Drageekern enthält:

| | |
|---|---|
| Wirksubstanz | 75,0 mg |
| Calciumphosphat | 93,0 mg |
| Maisstärke | 35,5 mg |
| Polyvinylpyrrolidon | 10,0 mg |
| Hydroxypropylmethylcellulose | 15,0 mg |
| Magnesiumstearat | 1,5 mg |
| | 230,0 mg |

### Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm-Maschenweite gerieben und mit der restlichen Menge Magnesiumstearat vermischt. Dieses Granulat wird auf einer Tablettiermaschine zu Tabletten mit der gewünschten Form gepreßt.

| | |
|---|---|
| Kerngewicht: | 230 mg |
| Stempel: | 9 mm, gewölbt |

Die so hergestellten Drageekerne werden mit einem Film überzogen, der im wesentlichen aus Hydroxypropylmethylcellulose besteht. Die fertigen Filmdragees werden mit Bienenwachs geglänzt.
Drageegewicht: 245 mg.

### Beispiel 7

### Tabletten mit 100 mg Wirksubstanz

### Zusammensetzung:

### 1 Tablette enthält:

| | |
|---|---|
| Wirksubstanz | 100,0 mg |
| Milchzucker | 80,0 mg |
| Maisstärke | 34,0 mg |
| Polyvinylpyrrolidon | 4,0 mg |
| Magnesiumstearat | 2,0 mg |
| | 220,0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2,0 mm-Maschenweite) und Trocknen im Hordentrockenschrank bei 50°C wird erneut gesiebt (1,5 mm-Maschenweite) und das Schmiermittel zugemischt. Die preßfertige Mischung wird zu Tabletten verarbeitet.

| | |
|---|---|
| Tablettengewicht: | 220 mg |
| Durchmesser: | 10 mm, biplan mit beidseitiger Facette und einseitiger Teilkerbe. |

### Beispiel 8

### Tabletten mit 150 mg Wirksubstanz

### Zusammensetzung:

| 1 Tablette enthält: | |
|---|---|
| Wirksubstanz | 150,0 mg |
| Milchzucker pulv. | 89,0 mg |
| Maisstärke | 40,0 mg |
| Kolloide Kieselgelsäure | 10,0 mg |
| Polyvinylpyrrolidon | 10,0 mg |
| Magnesiumstearat | 1,0 mg |
| | 300,0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1,5 mm-Maschenweite geschlagen.
Das bei 45°C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht: | 300 mg |
| Stempel: | 10 mm, flach |

### Beispiel 9

### Hartgelatine-Kapseln mit 150 mg Wirksubstanz

### 1 Kapsel enthält:

| | |
|---|---|
| Wirkstoff | 150,0 mg |
| Maisstärke getr. | ca. 180,0 mg |
| Milchzucker pulv. | ca. 87,0 mg |
| Magnesiumstearat | 3,0 mg |
| | ca. 420,0 mg |

### Herstellung:

Der Wirkstoff wird mit den Hilfsstoffen vermengt, durch ein Sieb von 0,75 mm-Maschenweite gegeben und in einem geeigneten Gerät homogen gemischt.
Die Endmischung wird in Hartgelatine-Kapseln der Größe 1 abgefüllt.
Kapselfüllung: ca. 320 mg
Kapselhülle: Hartgelatine-Kapsel Größe 1.

### Beispiel 10

### Suppositorien mit 150 mg Wirksubstanz

### 1 Zäpfchen enthält:

| | |
|---|---|
| Wirkstoff | 150,0 mg |
| Polyethylenglykol 1500 | 550,0 mg |
| Polyethylenglykol 6000 | 460,0 mg |
| Polyoxyethylensorbitanmonostearat | 840,0 mg |
| | 2000,0 mg |

### Herstellung:

Nach dem Aufschmelzen der Suppositorienmasse wird der Wirkstoff darin homogen verteilt und die Schmelze in vorgekühlte Formen gegossen.

### Beispiel 11

### Suspension mit 50 mg Wirksubstanz

### 100 ml Suspension enthalten:

| | |
|---|---|
| Wirkstoff | 1,00 g |
| Carboxymethylcellulose-Na-Salz | 0,10 g |
| p-Hydroxybenzoesäuremethylester | 0,05 g |
| p-Hydroxybenzoesäurepropylester | 0,01 g |
| Rohrzucker | 10,00 g |
| Glycerin | 5,00 g |
| Sorbitlösung 70%ig | 20,00 g |
| Aroma | 0,30 g |
| Wasser dest. | ad 100 ml |

### Herstellung:

Dest. Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.
5 ml Suspension enthalten 50 mg Wirkstoff.

### Beispiel 12

### Ampullen mit 10 mg Wirksubstanz

### Zusammensetzung:

| | |
|---|---|
| Wirkstoff | 10,0 mg |
| 0,01 n Salzsäure s.q. | |
| Aqua bidest | ad 2,0 ml |

### Herstellung:

Die Wirksubstanz wird in der erforderlichen Menge 0,01 n HCl gelöst, mit Kochsalz isotonisch gestellt, sterilfiltriert und in 2 ml Ampullen abgefüllt.

### Beispiel 13

### Ampullen mit 50 mg Wirksubstanz

### Zusammensetzung:

| | |
|---|---|
| Wirkstoff | 50,0 mg |
| 0,01 n Salzsäure s.q. | |
| Aqua bidest | ad 10,0 ml |

### Herstellung:

Die Wirksubstanz wird in der erforderlichen Menge 0,01 n HCl gelöst, mit Kochsalz isotonisch gestellt, sterilfiltriert und in 10 ml Ampullen abgefüllt.

## Patentansprüche

1. Verbindungen der allgemeinen Formel 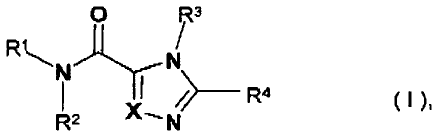 in der
R¹ eine Aryl-C₁₋₆-alkyl- oder Heteroaryl-C₁₋₆-alkylgruppe, in der jede Methylengruppe der Alkylgruppe durch ein oder zwei Fluoratome oder eine C₁₋₃-Alkylgruppe substituiert und eine Methylengruppe durch eine Carbonylgruppe ersetzt sein kann,
oder eine Aryl-C₂₋₆-alkenyl- oder Heteroaryl-C₂₋₆-alkenylgruppe, in denen die Alkenylkette mit 1 bis 10 Fluoratomen oder einer Cyan-, C₁₋₃-Alkyloxy-carbonyl-, C₁₋₃-Alkyl- oder Nitrogruppe substituiert sein kann,
R² ein Wasserstoffatom,
eine C₁₋₆-Alkylgruppe,
eine durch eine Gruppe Rₐ substituierte C₁₋₆-Alkylgruppe, wobei
Rₐ eine C₁₋₄-Alkoxy-carbonyl-gruppe bedeutet,
eine durch eine Gruppe R_{c} substituierte NH-Gruppe, wobei
R_{c} eine C₁₋₆-Alkyl-gruppe bedeutet,
eine Hydroxygruppe, oder
eine C₁₋₄-Alkoxygruppe,
X ein Stickstoffatom oder eine CH-Gruppe,
R³ eine 1-Buten-1-yl-, 2-Buten-1-yl-, 3-Methyl-2-buten-1-yl-, 2-Butin-1-yl-, Cyclopent-1-enyl-methyl-, Furanylmethyl-, Thienylmethyl-, Chlorbenzyl-, Brombenzyl-, lodbenzyl-, Methoxybenzyl- oder Cyanbenzylgruppe, und
R⁴ eine N-(2-Aminoethyl)-N-methyl-aminogruppe, die im Ethylteil durch ein oder zwei C₁₋₃-Alkylgruppen substituiert sein kann, oder
eine 3-Aminopiperidin-1-yl-, Piperazin-1-yl- oder [1,4]Diazepan-1-ylgruppe, wobei die vorstehend erwähnten Gruppen jeweils zusätzlich durch ein oder zwei C₁₋₃-Alkylgruppen substituiert sein können, bedeuten,
wobei unter den bei der Definition der vorstehend genannten Reste erwähnten Arylgruppen Phenyl- oder Naphthylgruppen zu verstehen sind, welche unabhängig voneinander durch Rₕ mono-, di- oder trisubstituiert sein können, wobei die Substituenten gleich oder verschieden sein können und Rₕ ein Fluor-, Chlor-, Brom- oder lodatom, eine Trifluormethyl-, Cyan-, Nitro-, Amino-, Aminocarbonyl-, C₁₋₃-Alkoxycarbonyl-, Aminosulfonyl-, Methylsulfonyl, Acetylamino-, Methylsulfonylamino-, C₁₋₃-Alkyl-, Cyclopropyl-, Ethenyl-, Ethinyl-, Morpholinyl-, Hydroxy-, C₁₋₃-Alkyloxy-, Difluormethoxy- oder Trifluomethoxygruppe darstellt, und in denen zusätzlich jedes Wasserstoffatom durch ein Fluoratom ersetzt sein kann,
unter den bei der Definition der vorstehend erwähnten Reste erwähnten Heteroarylgruppen eine Pyrrolyl-, Furanyl-, Thienyl-, Pyridyl-, Indolyl-, Benzofuranyl-, Benzothiophenyl-, Phenanthridinyl-, Chinolinyl- oder Isochinolinylgruppe zu verstehen ist, oder eine Pyrrolyl-, Furanyl-, Thienyl- oder Pyridylgruppe zu verstehen ist, in der eine oder zwei Methingruppen durch Stickstoffatome ersetzt sind,
oder eine Indolyl-, Benzofuranyl-, Benzothiophenyl-, Phenanthridinyl-, Chinolinyl- oder Isochinolinylgruppe zu verstehen ist, in der eine bis drei Methingruppen durch Stickstoffatome ersetzt sind,
oder eine 1,2-Dihydro-2-oxo-pyridinyl-, 1,4-Dihydro-4-oxo-pyridinyl-, 2,3-Dihydro-3-oxo-pyridazinyl-, 1,2,3,6-Tetrahydro-3,6-dioxo-pyridazinyl-, 1,2-Dihydro-2-oxo-pyrimi-dinyl-, 3,4-Dihydro-4-oxo-pyrimidinyl-, 1,2,3,4-Tetrahydro-2,4-dioxo-pyrimidinyl-, 1,2-Dihydro-2-oxo-pyrazinyl-, 1,2,3,4-Tetrahydro-2,3-dioxo-pyrazinyl-, 2,3-Dihydro-2-oxo-indolyl-, 2,3-Dihydrobenzofuranyl-, 2,3-Dihydro-2-oxo-1*H*-benzimidazolyl-, 2,3-Dihydro-2-oxo-benzoxazolyl-, 1,2-Dihydro-2-oxo-chinolinyl-, 1,4-Dihydro-4-oxo-chinolinyl-, 1,2-Dihydro-1-oxo-isochinolinyl-, 1,4-Dihydro-4-oxo-cinnolinyl-, 1,2-Dihydro-2-oxo-chinazolinyl-, 3,4-Dihydro-4-oxo-chinazolinyl-, 1,2,3,4-Tetrahydro-2,4-dioxo-chinazolinyl-, 1,2-Dihydro-2-oxochinoxalinyl-, 1,2,3,4-Tetrahydro-2,3-dioxo-chinoxalinyl-, 1,2-Dihydro-1-oxo-phthalazinyl-, 1,2,3,4-Tetrahydro-1,4-dioxo-phthalazinyl-, Chromanyl-, Cumarinyl-, 2,3-Dihydro-benzo[1,4]dioxinyl- oder 3,4-Dihydro-3-oxo-2*H*-benzo[1,4]oxazinyl-Gruppe zu verstehen ist,
und die vorstehend erwähnten Heteroarylgruppen durch Rₕ mono- oder disubstituiert sein können, wobei die Substituenten gleich oder verschieden sein können und Rₕ wie vorstehend erwähnt definiert ist,
deren Enantiomere, deren Diastereomere, deren Gemische und deren Salze.

2. Verbindungen der allgemeinen Formel I gemäß Anspruch 1, in denen
R¹ eine Phenylmethyl-, Phenylcarbonylmethyl-, Phenylprop-2-enyl-, Pyridinylmethyl-, Pyrimidinylmethyl-, Naphthylmethyl-, Chinolinylmethyl-, Isochinolinylmethyl-, Chinazolinylmethyl-, Chinoxalinylmethyl-, Phenanthridinylmethyl-, Naphthyridinylmethyl-oder Benzotriazolylmethylgruppe, wobei alle vorstehend genannten Aryl- und Heteroarylgruppen durch ein oder zwei Fluor-, Chlor- oder Bromatome oder ein oder zwei Cyan-, Nitro-, Amino-, C₁₋₃-Alkyl-, C₁₋₃-Alkyloxy- oder Morpholinylgruppen substituiert sein können, wobei die Substituenten gleich oder verschieden sind,
R² ein Wasserstoffatom oder eine Methylgruppe,
X ein Stickstoffatom oder eine CH-Gruppe,
R³ eine 1-Buten-1-yl-, 2-Buten-1-yl-, 3-Methyl-2-buten-1-yl-, 2-Butin-1-yl-, Cyclopent-1-enyl-methyl-, Furanylmethyl-, Thienylmethyl-, Benzyl-, Chlorbenzyl-, Brombenzyl-, Iodbenzyl- oder Cyanbenzylgruppe und
R⁴ eine N-(2-Aminoethyl)-N-methylamino-, N-(2-Aminopropyl)-N-methyl-amino-, 3-Aminopiperidin-1-yl-, Piperazin-1-yl- oder [1,4]Diazepan-1-ylgruppe bedeuten,
deren Enantiomere, deren Diastereomere, deren Gemische und deren Salze.

3. Verbindungen der allgemeinen Formel I gemäß Anspruch 2, in denen
R¹ eine Phenylethyl-, Naphthylmethyl-, Methylisochinolinylmethyl-, Chinolinylmethyl- oder Phenanthridinylmethylgruppe,
R² ein Wasserstoffatom oder eine Methylgruppe,
X ein Stickstoffatom oder eine CH-Gruppe,
R³ eine 3-Methyl-2-buten-1-yl-, 2-Butin-1-yl-, Benzyl- oder 2-Chlorbenzylgruppe und
R⁴ eine 3-Aminopiperidin-1-yl-, [1,4]Diazepan-1-yl- oder Piperazin-1-ylgruppe bedeuten,
deren Enantiomere, deren Diastereomere, deren Gemische und Salze.

4. Verbindungen gemäß einem der Ansprüche 1 bis 3, in denen R¹ bis R³ und X wie in einem der Ansprüche 1 bis 3 erwähnt definiert sind und R⁴ eine 3-Aminopiperidin-1-ylgruppe bedeutet, deren Tautomere, deren Enantiomere, deren Diastereomere, deren Gemische und Salze.

5. Verbindungen gemäß einem der Ansprüche 1 bis 4, in denen R¹ bis R⁴ wie in einem der Ansprüche 1 bis 5 erwähnt definiert sind und X ein Stickstoffatom bedeutet, deren Tautomere, deren Enantiomere, deren Diastereomere, deren Gemische und Salze.

6. Verbindungen gemäß einem der Ansprüche 1 bis 4, in denen R¹ bis R⁴ wie in einem der Ansprüche 1 bis 4 erwähnt definiert sind und X eine CH-Gruppe bedeutet, deren Tautomere, deren Enantiomere, deren Diastereomere, deren Gemische und Salze.

7. Folgende Verbindungen gemäß Anspruch 1:
(1) 1-(But-2-inyl)-2-(3-amino-piperidin-1-yl)-5-(naphth-1-ylmethylaminocarbonyl)-1H-imidazol
(2) 1-(But-2-inyl)-2-(3-amino-piperidin-1-yl)-5-[N-(naphth-1-ylmethyl)-N-methyl-aminocarbonyl]-1H-imidazol
(3) 1-(But-2-inyl)-2-(3-amino-piperidin-1-yl)-5-(phenanthridin-6-ylmethylamino-carbonyl)-1H-imidazol
(4) 1-(But-2-inyl)-2-(3-amino-piperidin-1-yl)-5-(3-methyl-isochinolin-1-ylmethylamino-carbonyl)-1H-imidazol
(5) 1-(But-2-inyl)-2-(3-amino-piperidin-1-yl)-5-(chinolin-3-ylmethylaminocarbonyl)-1H-imidazol
(6) 1-(But-2-inyl)-2-(3-amino-piperidin-1-yl)-5-(2-phenyl-ethylaminocarbonyl)-1H-imidazol
(7) 3-(Piperazin-1-yl)-4-(3-methyl-but-2-enyl)-5-(naphth-1-ylmethylaminocarbonyl)-4H-[1,2,4]triazol
(8) 3-(Piperazin-1-yl)-4-benzyl-5-(naphth-1-ylmethylaminocarbonyl)-4H-[1,2,4]triazol
(9) 3-([1,4]Diazepan-1-yl)-4-(3-methyl-but-2-enyl)-5-(naphth-1-ylmethylamino-carbonyl)-4H-[1,2,4]triazol
(10) 3-(3-Amino-piperidin-1-yl)-4-(3-methyl-but-2-enyl)-5-(naphth-1-ylmethylamino-carbonyl)-4H-[1,2,4]triazol
(11) 3-(3-Amino-piperidin-1-yl)-4-benzyl-5-(naphth-1-ylmethylaminocarbonyl)-4H-[1,2,4]triazol
(12) 3-(3-Amino-piperidin-1-yl)-4-(2-chlor-benzyl)-5-(naphth-1-ylmethylamino-carbonyl)-4H-[1,2,4]triazol
(13) 3-(3-Amino-piperidin-1-yl)-4-(but-2-inyl)-5-(3-methyl-isochinolin-1-ylmethyl-aminocarbonyl)-4H-[1,2,4]triazol
(14) 3-(3-Amino-piperidin-1-yl)-4-(but-2-inyl)-5-(chinolin-6-ylmethylaminocarbonyl)-4H-[1,2,4]triazol
(15) 3-(3-Amino-piperidin-1-yl)-4-(but-2-inyl)-5-(phenanthridin-6-ylmethylamino-carbonyl)-4H-[1,2,4]triazol
sowie deren Salze.

8. Physiologisch verträgliche Salze der Verbindungen gemäß den Ansprüchen 1 bis 7 mit anorganischen oder organischen Säuren oder Basen.

9. Arzneimittel, enthaltend eine Verbindung nach einem der Ansprüche 1 bis 7 oder ein physiologisch verträgliches Salz gemäß Anspruch 8 neben gegebenenfalls einem oder mehreren inerten Trägerstoffen und/oder Verdünnungsmitteln.

10. Verbindungen gemäß einem der Ansprüche 1 bis 8 zur Verwendung als Arzneimittel.

11. Verwendung einer Verbindung nach mindestens einem der Ansprüche 1 bis 8 zur Herstellung eines Arzneimittels, das zur Behandlung von Diabetes mellitus Typ I und Typ II, Arthritis, Adipositas, Allograft Transplantation und durch Calcitonin verursachte Osteoporose geeignet ist.

12. Verfahren zur Herstellung eines Arzneimittels gemäß Anspruch 9, **dadurch gekennzeichnet, daß** auf nichtchemischen Weg eine Verbindung nach mindestens einem der Ansprüche 1 bis 8 in einen oder mehrere inerte Trägerstoffe und/oder Verdünnungsmittel eingearbeitet wird.

13. Verfahren zu Herstellung der Verbindungen der allgemeinen Formel I gemäß Anspruch 1, **dadurch gekennzeichnet, daß**
a) zur Herstellung einer Verbindung der allgemeinen Formel I, in der X ein Stickstoff-atom ist: eine Verbindung der allgemeinen Formel 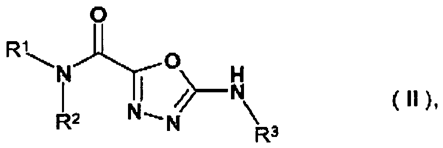 in der R¹ bis R³ wie in Anspruch 1 erwähnt definiert sind, mit einer Verbindung der allgemeinen Formel
H - R⁴ (III),
in der
R⁴ wie in Anspruch 1 erwähnt definiert ist, umgesetzt wird;
b) zur Herstellung einer Verbindung der allgemeinen Formel I, in der X eine CH-Gruppe ist: eine Verbindung der allgemeinen Formel 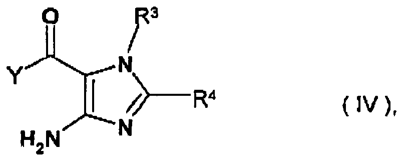
in der Y eine C₁₋₆-Alkoxy-, Di-(C₁₋₄-alkyl)-amino- oder -NR¹R²- Gruppe, in der R¹ wie in Anspruch 1 erwähnt definiert ist und R² mit Ausnahme des Wasserstoffatoms wie in Anspruch 1 erwähnt definiert ist, bedeutet und
R³ und R⁴ wie in Anspruch 1 erwähnt definiert sind,
diazotiert und anschließend reduziert wird und gegebenenfalls die Gruppe Y in die Gruppe -NR¹R², in der R¹ und R² wie in Anspruch 1 erwähnt definiert sind, umgewandelt wird;
c) Zur Herstellung einer Verbindung der allgemeinen Formel I, in der R⁴ gemäß der in Anspruch 1 erwähnten Definition eine Aminogruppe oder eine gegebenenfalls im Alkylteil substituierte Alkylaminogruppe enthält:
eine Verbindung der allgemeinen Formel 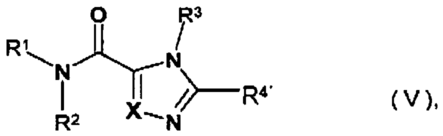
in der R¹, R², X und R³ wie in Anspruch 1 definiert sind und
R⁴' eine N-tert.-Butyloxycarbonylamino-, N-tert.-Butyloxycarbonyl-N-alkylamino-, Phthalimido- oder Azidogruppe enthält, wobei der Alkylteil der N-tert.-Butyloxy-carbonyl-N-alkylaminogruppe wie in Anspruch 1 erwähnt substituiert sein kann, entschützt wird;
und anschließend gegebenenfalls eine so erhaltene Verbindung der allgemeinen Formel I, die eine Amino-, Alkylamino- oder Iminogruppe enthält, mittels Acylierung oder Sulfonylierung in eine entsprechende Acyl- oder Sulfonylverbindung der allgemeinen Formel I übergeführt werden und/oder
eine so erhaltene Verbindung der allgemeinen Formel I, die eine Amino-, Alkyl-amino- oder Iminogruppe enthält, mittels Alkylierung oder reduktiver Alkylierung in eine entsprechende Alkylverbindung der allgemeinen Formel I übergeführt werden und/oder
eine so erhaltene Verbindung der allgemeinen Formel I, die eine Carboxygruppe enthält, mittels Veresterung in einen entsprechenden Ester der allgemeinen Formel I übergeführt werden und/oder
eine so erhaltene Verbindung der allgemeinen Formel I, die eine Carboxy- oder Estergruppe enthält, durch Umsetzung mit einem Amin in ein entsprechendes Amid der allgemeinen Formel I übergeführt werden, und/oder
während der Umsetzung verwendete Schutzgruppen abgespalten werden und/oder
die so erhaltenen Verbindungen der allgemeinen Formel I in ihre Enantiomeren und/oder Diastereomeren aufgetrennt werden und/oder
die erhaltenen Verbindungen der Formel I in ihre Salze, insbesondere für die pharmazeutische Anwendung in ihre physiologisch verträglichen Salze mit anorganischen oder organischen Säuren oder Basen, übergeführt werden.

## Claims

1. Compounds of general formula 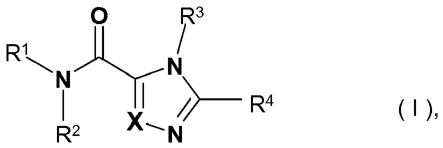 wherein
R¹ denotes an aryl-C₁₋₆-alkyl or heteroaryl-C₁₋₆-alkyl group wherein each methylene group of the alkyl group may be substituted by one or two fluorine atoms or a C₁₋₃-alkyl group and a methylene group may be replaced by a carbonyl group,
or an aryl-C₂₋₆-alkenyl or heteroaryl-C₂₋₆-alkenyl group, wherein the alkenyl chain may be substituted by 1 to 10 fluorine atoms or a cyano, C₁₋₃-alkyloxy-carbonyl, C₁₋₃-alkyl or nitro group,
R² denotes a hydrogen atom,
a C₁₋₆-alkyl group,
a C₁₋₆-alkyl group substituted by a group Rₐ, where
Rₐ denotes a C₁₋₄-alkoxy-carbonyl group,
an NH group substituted by a group R_{c}, wherein
R_{c} denotes a C₁₋₆-alkyl group,
a hydroxyl group, or
a C₁₋₄-alkoxy group,
X denotes a nitrogen atom or a CH group,
R³ denotes a 1-buten-1-yl, 2-buten-1-yl, 3-methyl-2-buten-1-yl, 2-butyn-1-yl, cyclopent-1-enyl-methyl, furanylmethyl, thienylmethyl, chlorobenzyl, bromobenzyl, iodobenzyl, methoxybenzyl or cyanobenzyl group, and
R⁴ denotes an N-(2-aminoethyl)-N-methyl-amino group which may be substituted in the ethyl moiety by one or two C₁₋₃-alkyl groups, or
a 3-aminopiperidin-1-yl, piperazin-1-yl or [1,4]diazepan-1-yl group, while the above-mentioned groups may each additionally be substituted by one or two C₁₋₃-alkyl groups,
while by the aryl groups mentioned in the definition of the above groups are meant phenyl or naphthyl groups, which may be mono-, di- or trisubstituted independently of one another by Rₕ, where the substituents may be identical or different and Rₕ denotes a fluorine, chlorine, bromine or iodine atom, a trifluoromethyl, cyano, nitro, amino, aminocarbonyl, C₁₋₃-alkoxy-carbonyl, aminosulphonyl, methylsulphonyl, acetylamino, methylsulphonylamino, C₁₋₃-alkyl, cyclopropyl, ethenyl, ethynyl, morpholinyl, hydroxy, C₁₋₃-alkyloxy, difluoromethoxy or trifluoromethoxy group, and wherein additionally each hydrogen atom may be replaced by a fluorine atom,
by the heteroaryl groups mentioned in the definition of the above-mentioned groups are meant a pyrrolyl, furanyl, thienyl, pyridyl, indolyl, benzofuranyl, benzothiophenyl, phenanthridinyl, quinolinyl or isoquinolinyl group,
or a pyrrolyl, furanyl, thienyl or pyridyl group, wherein one or two methyne groups are replaced by nitrogen atoms,
or an indolyl, benzofuranyl, benzothiophenyl, phenanthridinyl, quinolinyl or isoquinolinyl group, wherein one to three methyne groups are replaced by nitrogen atoms,
or a 1,2-dihydro-2-oxo-pyridinyl, 1,4-dihydro-4-oxo-pyridinyl, 2,3-dihydro-3-oxo-pyridazinyl, 1,2,3,6-tetrahydro-3,6-dioxo-pyridazinyl, 1,2-dihydro-2-oxo-pyrimidinyl, 3,4-dihydro-4-oxo-pyrimidinyl, 1,2,3,4-tetrahydro-2,4-dioxo-pyrimidinyl, 1,2-dihydro-2-oxo-pyrazinyl, 1,2,3,4-tetrahydro-2,3-dioxo-pyrazinyl, 2,3-dihydro-2-oxo-indolyl, 2,3-dihydrobenzofuranyl, 2,3-dihydro-2-oxo-1*H*-benzimidazolyl, 2,3-dihydro-2-oxo-benzoxazolyl, 1,2-dihydro-2-oxo-quinolinyl, 1,4-dihydro-4-oxo-quinolinyl, 1,2-dihydro-1-oxo-isoquinolinyl, 1,4-dihydro-4-oxo-cinnolinyl, 1,2-dihydro-2-oxo-quinazolinyl, 3,4-dihydro-4-oxo-quinazolinyl, 1,2,3,4-tetrahydro-2,4-dioxo-quinazolinyl, 1,2-dihydro-2-oxoquinoxalinyl, 1,2,3,4-tetrahydro-2,3-dioxo-quinoxalinyl, 1,2-dihydro-1-oxo-phthalazinyl, 1,2,3,4-tetrahydro-1,4-dioxo-phthalazinyl, chromanyl, cumarinyl, 2,3-dihydro-benzo[1,4]dioxinyl or 3,4-dihydro-3-oxo-2*H*-benzo[1,4]oxazinyl group,
and the above-mentioned heteroaryl groups may be mono- or disubstituted by Rₕ, while the substituents may be identical or different and Rₕ is as hereinbefore defined,
the enantiomers, diastereomers, the mixtures thereof and the salts thereof.

2. Compounds of general formula I according to claim 1, wherein
R¹ denotes a phenylmethyl, phenylcarbonylmethyl, phenylprop-2-enyl, pyridinylmethyl, pyrimidinylmethyl, naphthylmethyl, quinolinylmethyl, isoquinolinylmethyl, quinazolinylmethyl, quinoxalinylmethyl, phenanthridinylmethyl, naphthyridinylmethyl or benzotriazolylmethyl group, while all the above-mentioned aryl and heteroaryl groups may be substituted by one or two fluorine, chlorine or bromine atoms or one or two cyano, nitro, amino, C₁₋₃-alkyl, C₁₋₃-alkyloxy or morpholinyl groups, while the substituents are identical or different,
R² denotes a hydrogen atom or a methyl group,
X denotes a nitrogen atom or a CH group,
R³ denotes a 1-buten-1-yl, 2-buten-1-yl, 3-methyl-2-buten-1-yl, 2-butyn-1-yl, cyclopent-1-enyl-methyl, furanylmethyl, thienylmethyl, benzyl, chlorobenzyl, bromobenzyl, iodobenzyl or cyanobenzyl group and
R⁴ denotes an N-(2-aminoethyl)-N-methylamino, N-(2-aminopropyl)-N-methyl-amino, 3-aminopiperidin-1-yl, piperazin-1-yl or [1,4]diazepan-1-yl group,
the enantiomers, the diastereomers, the mixtures thereof and the salts thereof.

3. Compounds of general formula I according to claim 2, wherein
R¹ denotes a phenylethyl, naphthylmethyl, methylisoquinolinylmethyl, quinolinylmethyl or phenanthridinylmethyl group,
R² denotes a hydrogen atom or a methyl group,
X denotes a nitrogen atom or a CH group,
R³ denotes a 3-methyl-2-buten-1-yl, 2-butyn-1-yl, benzyl or 2-chlorobenzyl group and
R⁴ denotes a 3-aminopiperidin-1-yl, [1,4]diazepan-1-yl or piperazin-1-yl group, the enantiomers, the diastereomers, the mixtures thereof and salts.

4. Compounds according to one of claims 1 to 3, wherein R¹ to R³ and X are defined as in one of claims 1 to 3 and R⁴ denotes a 3-aminopiperidin-1-yl group, the tautomers, the enantiomers, the diastereomers, the mixtures and salts thereof.

5. Compounds according to one of claims 1 to 4, wherein R¹ to R⁴ are defined as in one of claims 1 to 5 and X denotes a nitrogen atom, the tautomers, the enantiomers, the diastereomers, the mixtures and salts thereof.

6. Compounds according to one of claims 1 to 4, wherein R¹ to R⁴ are defined as in one of claims 1 to 4 and X denotes a CH group, the tautomers, the enantiomers, the diastereomers, the mixtures and salts thereof.

7. The following compounds according to claim 1:
(1) 1-(but-2-ynyl)-2-(3-amino-piperidin-1-yl)-5-(naphth-1-ylmethylaminocarbonyl)-1 H-imidazole
(2) 1-(but-2-ynyl)-2-(3-amino-piperidin-1-yl)-5-[N-(naphth-1-ylmethyl)-N-methylaminocarbonyl]-1 H-imidazole
(3) 1-(but-2-ynyl)-2-(3-amino-piperidin-1-yl)-5-(phenanthridin-6-ylmethylamino-carbonyl)-1 H-imidazole
(4) 1-(but-2-ynyl)-2-(3-amino-piperidin-1-yl)-5-(3-methyl-isoquinolin-1-ylmethylaminocarbonyl)-1 H-imidazole
(5) 1-(but-2-ynyl)-2-(3-amino-piperidin-1-yl)-5-(quinolin-3-ylmethylaminocarbonyl)-1 H-imidazole
(6) 1-(but-2-ynyl)-2-(3-amino-piperidin-1-yl)-5-(2-phenyl-ethylaminocarbonyl)-1H-imidazole
(7) 3-(piperazin-1-yl)-4-(3-methyl-but-2-enyl)-5-(naphth-1-ylmethylaminocarbonyl)-4H-[1,2,4]triazole
(8) 3-(piperazin-1-yl)-4-benzyl-5-(naphth-1-ylmethylaminocarbonyl)-4H-[1,2,4]triazole
(9) 3-([1,4]diazepan-1-yl)-4-(3-methyl-but-2-enyl)-5-(naphth-1-ylmethylamino-carbonyl)-4H-[1,2,4]triazole
(10) 3-(3-amino-piperidin-1-yl)-4-(3-methyl-but-2-enyl)-5-(naphth-1-ylmethylaminocarbonyl)-4H-[1,2,4]triazole
(11) 3-(3-amino-piperidin-1-yl)-4-benzyl-5-(naphth-1-ylmethylaminocarbonyl)-4H-[1,2,4]triazole
(12) 3-(3-amino-piperidin-1-yl)-4-(2-chloro-benzyl)-5-(naphth-1-ylmethylamino-carbonyl)-4H-[1,2,4]triazole
(13) 3-(3-amino-piperidin-1-yl)-4-(but-2-ynyl)-5-(3-methyl-isoquinolin-1-ylmethyl-aminocarbonyl)-4H-[1,2,4]triazole
(14) 3-(3-amino-piperidin-1-yl)-4-(but-2-ynyl)-5-(quinolin-6-ylmethylaminocarbonyl)-4H-[1,2,4]triazole
(15) 3-(3-amino-piperidin-1-yl)-4-(but-2-ynyl)-5-(phenanthridin-6-ylmethylamino-carbonyl)-4H-[1,2,4]triazole
and the salts thereof.

8. Physiologically acceptable salts of the compounds according to claims 1 to 7 with inorganic or organic acids or bases.

9. Medicaments containing a compound according to one of claims 1 to 7 or a physiologically acceptable salt according to claim 8 optionally together with one or more inert carriers and/or diluents.

10. Compounds according to one of claims 1 to 8 for use as medicaments.

11. Use of a compound according to at least one of claims 1 to 8 for preparing a medicament which is suitable for the treatment of type I and type II diabetes mellitus, arthritis, obesity, allograft transplantation and osteoporosis caused by calcitonin.

12. Process for preparing a medicament according to claim 9, **characterised in that** a compound according to at least one of claims 1 to 8 is incorporated in one or more inert carriers and/or diluents by a non-chemical method.

13. Process for preparing the compounds of general formula I according to claim 1, **characterised in that**
a) in order to prepare a compound of general formula I wherein X is a nitrogen atom:
a compound of general formula 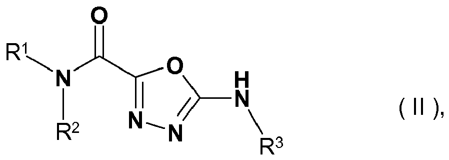 wherein R¹ to R³ are defined as in claim 1, is reacted with a compound of general formula
H - R⁴ (III),
wherein
R⁴ is defined as in claim 1;
b) in order to prepare a compound of general formula I wherein X is a CH group:
a compound of general formula 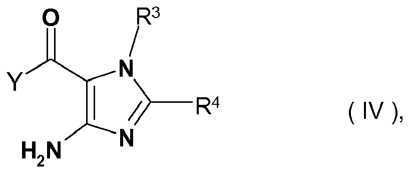 wherein Y denotes a C₁₋₆-alkoxy, di-(C₁₋₄-alkyl)-amino or -NR¹R² group wherein
R¹ is defined as in claim 1 and R² is defined as in claim 1 with the exception of the hydrogen atom, and
R³ and R⁴ are defined as in claim 1,
is diazotised and then reduced and optionally the group Y is converted into the group -NR¹R², wherein R¹ and R² are defined as in claim 1;
c) In order to prepare a compound of general formula I wherein R⁴ according to the definition given in claim 1 contains an amino group or an alkylamino group optionally substituted in the alkyl moiety:
a compound of general formula 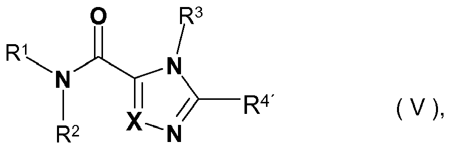
wherein R¹, R², X and R³ are defined as in claim 1 and
R^{4'} contains an N-tert.-butyloxycarbonylamino, N-tert.-butyloxycarbonyl-N-alkylamino, phthalimido or azido group, while the alkyl moiety of the N-tert.-butyloxycarbonyl-N-alkylamino group may be substituted as mentioned in claim 1, is deprotected;
and then optionally a compound of general formula I thus obtained which contains an amino, alkylamino or imino group, is converted by acylation or sulphonylation into a corresponding acyl or sulphonyl compound of general formula I and/or
a compound of general formula I thus obtained which contains an amino, alkylamino or imino group is converted by alkylation or reductive alkylation into a corresponding alkyl compound of general formula I and/or
a compound of general formula I thus obtained which contains a carboxy group is converted by esterification into a corresponding ester of general formula I, and/or
a compound of general formula I thus obtained which contains a carboxy or ester group is converted by reaction with an amine into a corresponding amide of general formula I, and/or
any protective groups used during the reaction are cleaved and/or
the compounds of general formula I thus obtained are resolved into their enantiomers and/or diastereomers and/or
the compounds of formula I obtained are converted into the salts thereof, particularly for pharmaceutical use into the physiologically acceptable salts thereof with inorganic or organic acids or bases.

## Revendications

1. Composés de formule générale 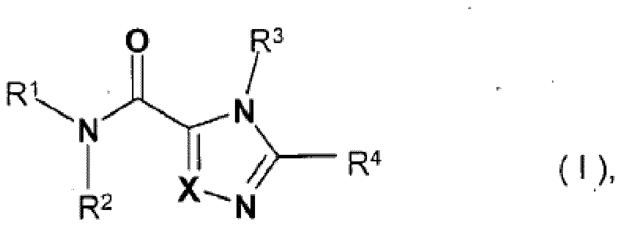 dans laquelle
R¹ représente un groupe aryl-alkyle en C₁ à C₆ ou hétéroaryl-alkyle en C₁ à C₆, dans lequel chaque groupe méthylène du groupe alkyle peut être substitué par un ou deux atomes de fluor ou un groupe alkyle en C₁ à C₃ et un groupe méthylène peut être remplacé par un groupe carbonyle,
ou un groupe aryl-alcényle en C₂ à C₆ ou hétéroaryl-alcényle en C₂ à C₆, dans lesquels la chaîne alcényle peut être substituée par 1 à 10 atomes de fluor ou un groupe cyano, alkyloxy en C₁ à C₃-carbonyle, alkyle en C₁ à C₃ ou nitro,
R² représente un atome d'hydrogène,
un groupe alkyle en C₁ à C₆,
un groupe alkyle en C₁ à C₆ substitué par un groupe Rₐ, où
Rₐ représente un groupe alcoxy en C₁ à C₄-carbonyle, un groupe NH substitué par un groupe R_{c}, où
R_{c} représente un groupe alkyle en C₁ à C₆,
un groupe hydroxy, ou
un groupe alcoxy en C₁ à C₄,
X représente un atome d'azote ou un groupe CH,
R³ représente un groupe 1-butén-1-yle, 2-butén-1-yle, 3-méthyl-2-butén-1-yle, 2-butin-1-yle, cyclopent-1-ényl-méthyle, furanylméthyle, thiénylméthyle, chlorobenzyle, bromobenzyle, iodobenzyle, méthoxybenzyle ou cyanobenzyle, et
R⁴ représente un groupe N-(2-aminoéthyl)-N-méthyl-amino qui peut être substitué dans la partie éthyle par un ou deux groupes alkyle en C₁ à C₃, ou
un groupe 3-aminopipéridin-1-yle, pipérazin-1-yle ou [1,4]diazépan-1-yle, où les groupes mentionnés précédemment peuvent être substitués en outre respectivement par un ou deux groupes alkyle en C₁ à C₃,
où les groupes aryle mentionnés dans la définition des radicaux cités précédemment désignent des groupes phényle ou naphtyle, qui peuvent être mono-, di- ou tri-substitués indépendamment les uns des autres par Rₕ, où les substituants peuvent être identiques ou différents et Rₕ représente un atome de fluor, de chlore, de brome ou d'iode, un groupe trifluorométhyle, cyano, nitro, amino, aminocarbonyle, alcoxy en C₁ à C₃-carbonyle, aminosulfonyle, méthylsulfonyle, acétylamino, méthylsulfonylamino, alkyle en C₁ à C₃, cyclopropyle, éthényle, éthinyle, morpholinyle, hydroxy, alkyloxy en C₁ à C₃, difluorométhoxy ou trifluorométhoxy, et dans lesquels de plus chaque atome d'hydrogène peut être remplacé par un atome de fluor,
les groupes hétéroaryle mentionnés dans la définition des radicaux cités précédemment désignent un groupe pyrrolyle, furanyle, thiényle, pyridyle, indolyle, benzofuranyle, benzothiophényle, phénanthridinyle, quinolinyle ou isoquinolinyle,
ou désignent un groupe pyrrolyle, furanyle, thiényle ou pyridyle, dans lequel un ou deux groupes méthine sont remplacés par des atomes d'azote,
ou désignent un groupe indolyle, benzofuranyle, benzothiophényle, phénanthridinyle, quinolinyle ou isoquinolinyle dans lequel un à trois groupes méthine sont remplacés par des atomes d'azote,
ou désignent un groupe 1,2-dihydro-2-oxo-pyridinyle, 1,4-dihydro-4-oxo-pyridinyle, 2,3-dihydro-3-oxo-pyridazinyle, 1,2,3,6-tétrahydro-3,6-dioxo-pyridazinyle, 1,2-dihydro-2-oxo-pyrimidinyle, 3,4-dihydro-4-oxo-pyrimidinyle, 1,2,3,4-tétrahydro-2,4-dioxo-pyrimidinyle, 1,2-dihydro-2-oxo-pyrazinyle, 1,2,3,4-tétrahydro-2,3-dioxo-pyrazinyle, 2,3-dihydro-2-oxo-indolyle, 2,3-dihydrobenzofuranyle, 2,3-dihydro-2-oxo-1*H*-benzimi-dazolyle, 2,3-dihydro-2-oxo-benzoxazolyle, 1,2-dihydro-2-oxo-quinolinyle, 1,4-dihydro-4-oxo-quinolinyle, 1,2-dihydro-1-oxo-isoquinolinyle, 1,4-dihydro-4-oxo-cinnolinyle, 1,2-dihydro-2-oxo-quinazolinyle, 3,4-dihydro-4-oxo-quinazolinyle, 1,2,3,4-tétrahydro-2,4-dioxo-quinazolinyle, 1,2-dihydro-2-oxo-quinoxalinyle, 1,2,3,4-tétrahydro-2,3-dioxo-quinoxalinyle, 1,2-dihydro-1-oxo-phtalazinyle, 1,2,3,4-tétrahydro-1,4-dioxo-phtalazinyle, chromanyle, cumarinyle, 2,3-dihydro-benzo[1,4]dioxinyle ou 3,4-dihydro-3-oxo-2*H-*benzo[1,4]oxazinyle,
et les groupes hétéroaryle précédemment cités peuvent être mono- ou disubstitués par Rₕ, où les substituants peuvent être identiques ou différents et Rₕ est défini tel que mentionné précédemment, leurs énantiomères, leurs diastéréomères, leurs mélanges et leurs sels.

2. Composés de formule générale 1 selon la revendication 1, dans lesquels
R¹ représente un groupe phénylméthyle, phénylcarbonyl-méthyle, phénylprop-2-ényle, pyridinyl-méthyle, pyrimidinylméthyle, naphtylméthyle, quinolinylméthyle, isoquinolinylméthyle, quinazolinylméthyle, quinoxa-linylméthyle, phénanthridinylméthyle, naphtyridinyl-méthyle ou benzotriazolylméthyle, où tous les groupes aryle et hétéroaryle cités précédemment peuvent être substitués par un ou deux atomes de fluor, de chlore ou de brome ou un ou deux groupes cyano, nitro, amino, alkyle en C₁ à C₃, alkyloxy en C₁ à C₃ ou morpholinyle, où les substituants sont identiques ou différents,
R² représente un atome d'hydrogène ou un groupe méthyle,
X représente un atome d'azote ou un groupe CH,
R³ représente un groupe 1-butén-1-yle, 2-butén-1-yle, 3-méthyl-2-butén-1-yle, 2-butin-1-yle, cyclopent-1-ényl-méthyle, furanylméthyle, thiénylméthyle, benzyle, chlorobenzyle, bromobenzyle, iodobenzyle ou cyanobenzyle, et
R⁴ représente un groupe N-(2-aminoéthyl)-N-méthyl-amino, N-(2-aminopropyl)-N-méthyl-amino, 3-aminopipéridin-1-yle, pipérazin-1-yle ou [1,4]diazépan-1-yle,
leurs énantiomères, leurs diastéréomères, leurs mélanges et leurs sels.

3. Composés de formule générale I selon la revendication 2, dans lesquels
R¹ représente un groupe phényléthyle, naphtylméthyle, méthylisoquinolinylméthyle, quinolinylméthyle ou phénanthridinylméthyle,
R² représente un atome d'hydrogène ou un groupe méthyle,
X représente un atome d'azote ou un groupe CH,
R³ représente un groupe 3-méthyl-2-butén-1-yle, 2-butin-1-yle, benzyle ou 2-chlorobenzyle et
R⁴ représente un groupe 3-aminopipéridin-1-yle, [1,4]diazépan-1-yle ou pipérazin-1-yle,
leurs énantiomères, leurs diastéréomères, leurs mélanges et leurs sels.

4. Composés selon l'une des revendications 1 à 3, dans lesquels R¹ à R³ et X sont définis tels que mentionnés dans l'une des revendications 1 à 3 et R⁴ représente un groupe 3-aminopipéridin-1-yle, leurs tautomères, leurs énantiomères, leurs diastéréomères, leurs mélanges et leurs sels.

5. Composés selon l'une des revendications 1 à 4, dans lesquels R¹ à R⁴ sont définis tels que mentionnés dans l'une des revendications 1 à 5 et X représente un atome d'azote, leurs tautomères, leurs énantiomères, leurs diastéréomères, leurs mélanges et leurs sels.

6. Composés selon l'une des revendications 1 à 4, dans lesquels R¹ à R⁴ sont définis tels que mentionnés dans l'une des revendications 1 à 4 et X représente un groupe CH, leurs tautomères, leurs énantiomères, leurs diastéréomères, leurs mélanges et leurs sels.

7. Composés suivants selon la revendication 1 :
(1) 1-(but-2-inyl)-2-(3-amino-pipéridin-1-yl)-5-(napht-1-ylméthylaminocarbonyl)-1H-imidazol
(2) 1-(but-2-inyl)-2-(3-amino-pipéridin-1-yl)-5-[N-(napht-1-ylméthyl)-N-méthyl-aminocarbonyl]-1H-imidazol
(3) 1-(but-2-inyl)-2-(3-amino-pipéridin-1-yl)-5-(phénanthridin-6-ylméthylaminocarbonyl)-1H-imidazol
(4) 1-(but-2-inyl)-2-(3-amino-pipéridin-1-yl)-5-(3-méthyl-isoquinolin-1-ylméthylaminocarbonyl)-1H-imidazol
(5) 1-(but-2-inyl)-2-(3-amino-pipéridin-1-yl)-5-(quinolin-3-ylméthylaminocarbonyl)-1H-imidazol
(6) 1-(but-2-inyl)-2-(3-amino-pipéridin-1-yl)-5-(2-phényl-éthylaminocarbonyl)-1H-imidazol
(7) 3-(pipérazin-1-yl)-4-(3-méthyl-but-2-ényl)-5-(napht-1-ylméthylaminocarbonyl)-4H-[1,2,4]triazol
(8) 3-(pipérazin-1-yl)-4-benzyl-5-(napht-1-ylméthylaminocarbonyl)-4H-[1,2,4]triazol
(9) 3-([1,4]diazépan-1-yl)-4-(3-méthyl-but-2-ényl)-5-(napht-1-ylméthylaminocarbonyl)-4H-[1,2,4]triazol
(10) 3-(3-amino-pipéridin-1-yl)-4-(3-méthyl-but-2-ényl)-5-(napht-1-ylméthylaminocarbonyl)-4H-[1,2,4]triazol
(11) 3-(3-amino-pipéridin-1-yl)-4-benzyl-5-(napht-1-ylméthylaminocarbonyl)-4H-[1,2,4]triazol
(12) 3-(3-amino-pipéridin-1-yl)-4-(2-chloro-benzyl)-5-(napht-1-ylméthylaminocarbonyl)-4H-[1,2,4]triazol
(13) 3-(3-amino-pipéridin-1-yl)-4-(but-2-inyl)-5-(3-méthyl-isoquinolin-1-ylméthylaminocarbonyl)-4H-[1,2,4]triazol
(14) 3-(3-amino-pipéridin-1-yl)-4-(but-2-inyl)-5-(quinolin-6-ylméthylaminocarbonyl)-4H-[1,2,4]triazol
(15) 3-(3-amino-pipéridin-1-yl)-4-(but-2-inyl)-5-(phénanthridin-6-ylméthylaminocarbonyl)-4H-[1,2,4]triazol
et leurs sels.

8. Sels physiologiquement acceptables des composés selon les revendications 1 à 7, avec des acides ou des bases inorganiques ou organiques.

9. Médicament contenant un composé selon l'une des revendications 1 à 7 ou un sel physiologiquement acceptable selon la revendication 8, outre éventuellement un ou plusieurs véhicules et/ou diluants inertes.

10. Composés selon l'une des revendications 1 à 8, pour leur utilisation comme médicament.

11. Utilisation d'un composé selon au moins l'une des revendications 1 à 8, pour la production d'un médicament qui est approprié pour le traitement du diabète sucré de type I et de type II, de l'arthrite, de l'adiposité, pour une allogreffe et pour l'ostéoporose due à la calcitonine.

12. Procédé de production d'un médicament selon la revendication 9, **caractérisé en ce que**, de façon non chimique, un composé selon au moins l'une des revendications 1 à 8 est incorporé dans un ou plusieurs véhicules inertes et/ou diluants.

13. Procédé de production des composés de formule générale I selon la revendication 1, **caractérisé en ce que**
a) pour la production d'un composé de formule générale I, dans laquelle X est un atome d'azote : on fait réagir un composé de formule générale 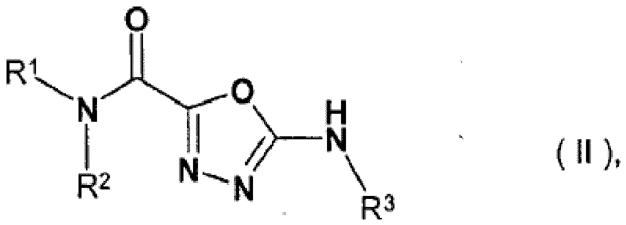 dans laquelle R¹ à R³ sont définis tels que mentionnés dans la revendication 1, avec un composé de formule générale
**H - R⁴** **(III),**
dans laquelle
R⁴ est défini tel que mentionné dans la revendication 1 ;
b) pour la production d'un composé de formule générale I, dans laquelle X est un groupe CH : un composé de formule générale 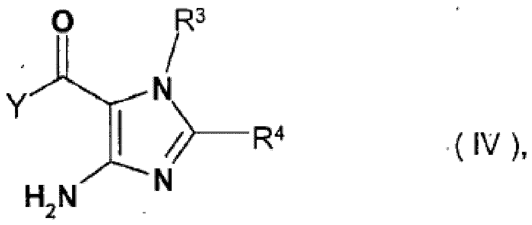
dans laquelle Y représente un groupe alcoxy en C₁ à C₆, di- (alkyle en C₁ à C₄) -amino ou -NR¹R² dans lequel R¹ est défini tel que mentionné dans la revendication 1 et R², à l'exception de l'atome d'hydrogène, est défini tel que mentionné dans la revendication 1, et
R³ et R⁴ sont définis tels que mentionnés dans la revendication 1,
est diazoté et est ensuite réduit, et éventuellement le groupe Y est transformé en groupe -NR¹R², dans lequel R¹ et R² sont définis tels que mentionnés dans la revendication 1 ;
c) pour la production d'un composé de formule générale I dans laquelle R⁴ contient selon la définition mentionnée dans la revendication 1 un groupe amino ou un groupe alkylamino éventuellement substitué dans la partie alkyle :
un composé de formule générale 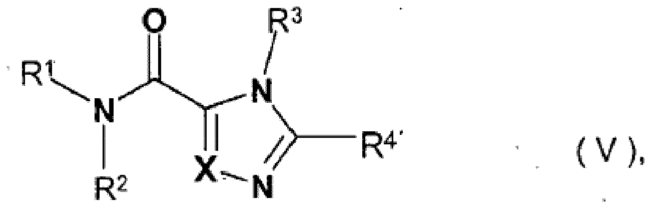
dans laquelle R¹, R², X et R³ sont tels que définis dans la revendication 1 et
R^{4'} contient un groupe N-tert-butyloxycarbonylamino, N-tert-butyloxycarbonyl-N-alkylamino, phtalimido ou azido,
où la partie alkyle du groupe N-tert-butyloxycarbonyl-N-alkylamino peut être substituée comme mentionné dans la revendication 1,
est déprotégé ;
et ensuite éventuellement, un composé de formule générale I ainsi obtenu, qui contient un groupe amino, alkylamino ou imino, sont convertis par acylation ou sulfonylation en un composé acyle ou sulfonyle de formule générale I correspondant et/ou
un composé de formule générale I ainsi obtenu, qui contient un groupe amino, alkylamino ou imino, sont convertis par alkylation ou alkylation réductrice en un composé alkyle de formule générale I correspondant et/ou
un composé de formule générale I ainsi obtenu, qui contient un groupe carboxy, sont convertis par estérification en un ester de formule générale I correspondant et/ou
un composé de formule générale I ainsi obtenu, qui contient un groupe carboxy ou ester, sont convertis par réaction avec une amine en un composé amide de formule générale I correspondant et/ou
pendant la réaction, des groupes protecteurs utilisés sont clivés et/ou
les composés de formule générale I ainsi obtenus sont séparés en leurs énantiomères et/ou diastéréomères et/ou
les composés de formule I obtenus sont convertis en leurs sels, en particulier pour l'utilisation pharmaceutique, en leurs sels physiologiquement acceptables avec des acides ou des bases inorganiques ou organiques.
